# EUROPÄISCHE PATENTANMELDUNG

(11) **EP 1 982 772 A1**
(43) Veröffentlichungstag der Anmeldung: **22.10.2008**
(21) Anmeldenummer: 08104285.5
(22) Anmeldetag: 10.05.2004
(51) Int. Cl.: B05D 7/24, C23C 18/12, C09D 201/00, A61L 31/08

(54) **Biokompatibel beschichtete medizinische Implantate**

(30) Priorität: 16.05.2003 DE 10322182; 28.05.2003 DE 10324415; 21.07.2003 DE 10333098
(62) Teilanmeldung aus: 04731916.5
(71) Anmelder: CINVENTION AG, 65203 Wiesbaden (DE)
(72) Erfinder: Rathenow, Jörg, 49610 Quakenbrück (DE); Ban, Andreas, 64285 Darmstadt (DE); Kunstmann, Jürgen, 65812 Bad Soden (DE); Mayer, Bernhard, 63071 Offenbach am Main (DE); Asgari, Sohéil, 65193 Wiesbaden (DE)
(74) Vertreter: Hansen, Norbert

(57) **Zusammenfassung**

Es werden implantierbare medizinische Vorrichtungen mit biokompatiblen Beschichtungen sowie Verfahren zu deren Herstellung beschrieben. Insbesondere betrifft die vorliegende Erfindung mit einer kohlenstoffhaltigen Schicht beschichtete medizinische implantierbare Vorrichtungen, die durch mindestens partielles Beschichten der Vorrichtung mit einem Polymerfilm und Erhitzen des Polymerfilms in einer Atmosphäre, die im Wesentlichen frei von Sauerstoff ist, auf Temperaturen im Bereich von 200°C bis 2500°C hergestellt werden, wobei eine kohlenstoffhaltige Schicht auf der implantierbaren medizinischen Vorrichtung erzeugt wird.

## Beschreibung

Die vorliegende Erfindung betrifft implantierbare medizinische Vorrichtungen mit biokompatiblen Beschichtungen sowie ein Verfahren zu deren Herstellung. Insbesondere betrifft die vorliegende Erfindung mit einer kohlenstoffhaltigen Schicht beschichtete medizinische implantierbare Vorrichtungen, die durch mindestens partielles Beschichten der Vorrichtung mit einem Polymerfilm und Erhitzen des Polymerfilms in einer Atmosphäre, die im Wesentlichen frei von Sauerstoff ist, auf Temperaturen im Bereich von 200°C bis 2500°C erhältlich sind, wobei eine kohlenstoffhaltige Schicht auf der implantierbaren medizinischen Vorrichtung erzeugt wird.

Medizinische Implantate wie chirurgische bzw. orthopädische Schrauben, Platten, Gelenkprothesen, künstliche Herzklappen, Gefäßprothesen, Stents, als auch subkutan oder intramuskulär implantierbare Wirkstoffdepots werden aus verschiedenartigsten Materialien, die nach den spezifischen biochemischen und mechanischen Eigenschaften ausgewählt werden, hergestellt. Diese Materialien müssen für den dauerhaften Einsatz im Körper geeignet sein, keine toxischen Stoffe freisetzen und bestimmte mechanische und biochemische Eigenschaften aufweisen.

Die beispielsweise für Stents und Gelenkprothesen häufig verwendeten Metalle oder Metalllegierungen, sowie keramischen Materialien weisen jedoch häufig, insbesondere beim Dauereinsatz, Nachteile hinsichtlich ihrer Biokompatibilität auf. Implantate lösen durch chemische und/oder physikalische Reizung unter Anderem inflammatorische Gewebe- und Immunreaktionen aus, so dass es zu Unverträglichkeitsreaktionen im Sinne von chronischen Entzündungsreaktionen mit Abwehr- und Abstoßungsreaktionen, überschießender Narbenbildung oder Gewebeabbau kommt, die im Extremfall dazu führen müssen, dass das Implantat entfernt und ersetzt werden muss oder aber zusätzliche therapeutische Interventionen invasiver oder nichtinvasiver Art angezeigt sind.

Mangelnde Verträglichkeit mit dem umliegenden Gewebe führt beispielsweise bei Koronarstents zu hohen Restenoseraten, da einerseits die Intima der Gefäßwand zu entzündungsbedingter Makrophagenreaktion mit Narbenbildung neigt, und andererseits sowohl die direkten Oberflächeneigenschaften als auch die pathologisch veränderte Gefäßwand im Gebiet des Stents zu Aggregation von Thrombozyten an dem Gefäßimplantat selbst, sowie auf entzündlich veränderten Gefäßwänden führt. Beide Mechanismen unterhalten einen sich wechselseitig beeinflussenden Entzündungs- und Unverträglichkeitsprozess, der in 20 - 30 % der interventionell mit Stents versorgten Patienten zu einer behandlungsbedürftigen erneuten Verengung der Koronararterie führt.

Aus diesem Grund gab es im Stand der Technik verschiedene Ansätze, die Oberflächen medizinischer Implantate in geeigneter Weise zu beschichten, um die Biokompatibilität der verwendeten Materialien zu erhöhen und Abwehr- bzw. Abstoßungsreaktionen zu verhindern.
In der US 5,891,507 werden beispielsweise Verfahren zur Beschichtung der Oberfläche von Metallstents mit Silikon, Polytetrafluorethylen sowie biologischen Materialien wie Heparin oder Wachstumsfaktoren beschrieben, welche die Bioverträglichkeit der Metallstents erhöhen.

Neben Kunststoffschichten haben sich kohlenstoffbasierte Schichten als besonders vorteilhaft erwiesen.

So sind beispielsweise aus der DE 199 51 477 Koronarstents mit einer Beschichtung aus amorphem Siliziumkarbid bekannt, welches die Biokompatibilität des Stentmaterials erhöht. Das US-Patent 6,569,107 beschreibt kohlenstoffbeschichtete Stents, bei welchen das Kohlenstoffmaterial mittels chemischer oder physikalischer Dampfphasenabscheidungs-methoden (CVD oder PVD) aufgebracht wurde. Auch im US-Patent 5,163,958 werden rohrförmige Endoprothesen oder Stents mit einer kohlenstoffbeschichteten Oberfläche beschrieben, die antithrombogene Eigenschaften aufweist. Die WO 02/09791 beschreibt Intravaskularstents mit Beschichtungen die durch CVD von Siloxanen erzeugt werden.

Die Abscheidung von pyrolytischem Kohlenstoff unter PVD- oder CVD-Bedingungen erfordert die sorgfältige Auswahl geeigneter gasförmiger oder verdampfbarer Kohlenstoffpräkursoren, die dann oft bei hohen Temperaturen, zum Teil unter Plasmabedingungen, in einer Inertgas- oder Hochvakuumatmosphäre auf das Implantat abgeschieden werden.

Neben den CVD-Verfahren zur Abscheidung von Kohlenstoff werden im Stand der Technik verschiedene Sputterverfahren im Hochvakuum zur Herstellung pyrolytischer Kohlenstoffschichten mit verschiedener Struktur beschrieben, siehe hierzu beispielsweise die US 6,355,350.

All diesen Verfahren des Standes der Technik ist gemeinsam, dass die Abscheidung von Kohlenstoffsubstraten unter zum Teil extremen Temperatur- und/oder Druckbedingungen sowie mittels Anwendung aufwendiger Prozesssteuerung stattfindet.

Ein weiterer Nachteil der Verfahren des Standes der Technik liegt darin, dass aufgrund unterschiedlicher thermischer Ausdehnungseffizienten von Materialien, aus welchen die Implantate gefertigt sind, und den aufgebrachten CVD-Schichten oft nur eine geringe Haftung der Schicht auf dem Implantat erzielt wird, wodurch es zu Abplatzungen, Rissen und Verschlechterung der Oberflächenqualität kommt, welche sich nachteilig auf die Verwendbarkeit der Implantate auswirken.

Es besteht daher ein Bedarf nach einfach anwendbaren und kostengünstigen Verfahren zur Beschichtung von implantierbaren medizinischen Vorrichtungen mit einem kohlenstoff-basierten Material, die in der Lage sind, biokompatible Oberflächenbeschichtungen aus kohlenstoffhaltigem Material zur Verfügung zu stellen.

Ferner besteht ein Bedarf nach kostengünstig herzustellenden biokompatibel beschichteten medizinischen Implantaten mit verbesserten Eigenschaften.

Eine Aufgabe der vorliegenden Erfindung ist es daher, ein Verfahren zur Herstellung biokompatibler Beschichtungen auf implantierbaren medizinischen Vorrichtungen zur Verfügung zu stellen, das mit kostengünstigen und in ihren Eigenschaften vielfältig variierbaren Ausgangsmaterialien auskommt und einfach steuerbare Verarbeitungs-bedingungen anwendet.

Eine weitere Aufgabe der vorliegenden Erfindung ist es, mit kohlenstoffhaltigen Beschichtungen versehene implantierbare medizinische Vorrichtungen zur Verfügung zu stellen, die eine erhöhte Bioverträglichkeit bzw. Biokompatibilität aufweisen.

Eine weitere Aufgabe der vorliegenden Erfindung ist es, biokompatibel beschichtete medizinische Implantate bereit zu stellen, deren Beschichtung die Aufbringung von medizinischen Wirkstoffen auf oder in die Implantatoberfläche ermöglicht.

Eine wiederum weitere Aufgabe der vorliegenden Erfindung ist es, beschichtete medizinische Implantate zur Verfügung zu stellen, welche aufgebrachte pharmakologisch wirksame Stoffe nach dem Einsetzen des Implantats in den menschlichen Körper gezielt und gegebenenfalls kontrolliert freisetzen können.

Eine weitere Aufgabe der Erfindung ist es, implantierbare Wirkstoffdepots mit einer Beschichtung bereitzustellen, welche die Freisetzung von Wirkstoffen aus dem Depot steuern kann.

Die erfindungsgemäße Lösung der oben genannten Aufgaben besteht in einem Verfahren sowie damit erhältlichen beschichteten medizinischen Implantaten, wie in den unabhängigen Ansprüchen definiert. Bevorzugte Ausführungsformen des erfindungsgemäßen Verfahrens bzw. der erfindungsgemäßen Erzeugnisse ergeben sich aus den abhängigen Unteransprüchen.

Im Rahmen der vorliegenden Erfindung wurde gefunden, dass sich kohlenstoffhaltige Schichten auf implantierbaren medizinischen Vorrichtungen unterschiedlichster Art auf einfache und reproduzierbare Weise dadurch herstellen lassen, dass die Vorrichtung zunächst mindestens teilweise mit einem Polymerfilm beschichtet wird, der anschließend in einer im Wesentlichen sauerstofffreien Atmosphäre bei hohen Temperaturen karbonisiert bzw. pyrolysiert wird. Bevorzugt ist, dass die resultierende(n) kohlenstoffhaltige(n) Schicht(en) nachfolgend mit Wirkstoffen, Mikroorganismen oder lebenden Zellen beladen werden. Ferner kann alternativ oder zusätzlich mit biologisch abbaubaren bzw. resorbierbaren Polymeren oder nicht-biologisch abbaubaren bzw. resorbierbaren Polymeren zumindest teilweise beschichtet werden.

Entsprechend umfasst das erfindungsgemäße Verfahren zur Herstellung biokompatibler Beschichtungen auf implantierbaren, medizinischen Vorrichtungen die folgenden Schritte:
a) mindestens partielles Beschichten der medizinischen Vorrichtung mit einem Polymerfilm mittels eines geeigneten Beschichtungs- bzw. Auftragungsverfahrens;
b) Erhitzen des Polymerfilms in einer Atmosphäre, die im Wesentlichen frei von Sauerstoff ist, bei Temperaturen im Bereich von 200°C bis 2500°C,
zur Erzeugung einer kohlenstoffhaltigen Schicht auf der medizinischen Vorrichtung.

Unter Karbonisierung oder auch Pyrolyse wird im Rahmen der vorliegenden Erfindung die partielle thermische Zersetzung oder Verkokung kohlenstoffhaltiger Ausgangsverbindungen verstanden, die in der Regel Oligo- oder Polymermaterialien auf Kohlenwasserstoffbasis sind, welche nach der Karbonisierung in Abhängigkeit von den gewählten Temperatur- und Druckbedingungen und der Art des verwendeten Polymermaterials kohlenstoffhaltige Schichten zurücklassen, die in ihrer Struktur von amorph bis zu hochgeordnet kristallinen graphitartigen Strukturen, sowie in ihrer Porosität und den Oberflächeneigenschaften exakt eingestellt werden können.

Das erfindungsgemäße Verfahren läßt sich nicht nur zur Beschichtung implantierbarer medizinischer Vorrichtungen, sondern in seinem allgemeinsten Aspekt auch generell zur Erzeugung kohlenstoffhaltiger Beschichtungen auf Substraten beliebiger Art verwenden. Die im folgenden im Hinblick auf Implantate als Substrat gemachten Aussagen gelten daher ausnahmslos auch für sonstige Substrate für andere Zwecke.

### IMPLANTATE

Mit dem erfindungsgemäßen Verfahren können biokompatible kohlenstoffhaltige Beschichtungen auf implantierbaren medizinischen Vorrichtungen aufgebracht werden.

Die Begriffe "implantierbare, medizinische Vorrichtung" und "Implantat" werden im weiteren synonym verwendet und umfassen medizinische oder therapeutische Implantate wie beispielsweise Gefäßendoprothesen, intraluminale Endoprothesen, Stents, Koronarstents, periphere Stents, chirurgische bzw. orthopädische Implantate für temporäre Zwecke wie chirurgische Schrauben, Platten, Nägel und sonstige Befestigungsmittel, permanente chirurgische oder orthopädische Implantate wie Knochen- oder Gelenkprothesen, beispielsweise künstliche Hüft- oder Kniegelenke, Gelenkpfanneneinsätze, Schrauben, Platten, Nägel, implantierbare orthopädische Fixierungshilfsmittel, Wirbelkörperersatzmittel, sowie Kunstherzen und Teile davon, künstliche Herzklappen, Herzschrittmachergehäuse, Elektroden, subkutane und/oder intramuskulär einsetzbare Implantate, Wirkstoffdepots und Mikrochips, und dergleichen.

Die mit dem Verfahren der vorliegenden Erfindung biokompatibel beschichtbaren Implantate können aus nahezu beliebigen, vorzugsweise im wesentlichen temperaturstabilen Materialien bestehen, insbesondere aus allen Materialien, aus denen Implantate hergestellt werden.

Beispiele hierfür sind amorpher und/oder (teil-)kristalliner Kohlenstoff, Vollkarbonmaterial, poröser Kohlenstoff, Graphit, Kohlenstoffverbundmaterialien, Kohlefasern, Keramiken wie z. B. Zeolithe, Silikate, Aluminiumoxide, Aluminosilikate, Siliziumkarbid, Siliziumnitrid; Metallkarbide, Metalloxide, Metallnitride, Metallcarbonitride, Metalloxycarbide, Metalloxynitride und Metalloxycarbonitride der Übergangsmetalle wie Titan, Zirkonium, Hafnium, Vanadium, Niob, Tantal, Chrom, Molybdän, Wolfram, Mangan, Rhenium, Eisen, Kobalt, Nickel; Metalle und Metalllegierungen, insbesondere der Edelmetalle Gold, Silber, Ruthenium, Rhodium, Palladium, Osmium, Iridium, Platin; Metalle und Metalllegierungen von Titan, Zirkon, Hafnium, Vanadin, Niob, Tantal, Chrom, Molybdän, Wolfram, Mangan, Rhenium, Eisen, Kobalt, Nickel, Kupfer; Stahl, insbesondere rostfreier Stahl, Formgedächtnislegierungen wie Nitinol, Nickel-Titanlegierung, Glas, Stein, Glasfasern, Mineralien, natürliche oder synthetische Knochensubstanz, Knochenimitate auf Basis von Erdalkalimetallkarbonaten wie Kalziumkarbonat, Magnesiumkarbonat, Strontiumkarbonat, sowie beliebige Kombinationen der genannten Materialien.

Daneben können auch Materialien beschichtet werden, die erst unter den Karbonisierungsbedingungen in ihre Endform umgewandelt werden. Beispiele hierfür sind Formkörper aus Papier, Fasermaterialien und polymeren Materialen, die nach Beschichtung mit dem Polymerfilm zusammen mit diesem zu beschichteten Kohlenstoffimplantaten umgewandelt werden.

Im erfindungsgemäßen Verfahren ist ferner die Herstellung beschichteter Implantate grundsätzlich auch ausgehend von keramischen Vorstufen des Implantats wie beispielsweise Keramikgrünkörpern möglich, die nach Beschichtung mit dem Polymerfilm zusammen mit der Karbonisierung des Polymerfilms zu ihrer endgültigen Anwendungsform ausgehärtet oder gesintert werden können. So können z.B. handelsübliche bzw. herkömmliche Keramiken (Bornitrid, Siliziumcarbid, etc.) oder auch nanokristalline Grünkörper aus Zirkoniumoxid und alpha- oder gamma-Al₂O₃ , oder gepresstes amorphes nanoskaliges ALOOH-Aerogel, welche zu nanoporösen kohlenstoffbeschichteten Formkörpern bei Temperaturen von etwa 500 - 2000°, bevorzugt jedoch etwa 800°C führen verwendet werden, wobei Beschichtungen mit Porositäten von etwa 10 - 100 nm erhalten werden können. Bevorzugte Anwendungsgebiete hierzu sind z.B. Vollimplantate zur Rekonstruktion von Gelenken, welche eine verbesserte Biokompatibilität aufweisen und zu einem homogenen Schichtenverbund führen.

Das erfindungsgemäße Verfahren löst das Problem von Delaminierungen von beschichteten Keramikimplantaten, welche unter biomechanischen Torsions- Zug-und Dehnungsbelastungen gewöhnlich zu Abrieb von sekundär aufgebrachten Beschichtungen neigen.

Die erfindungsgemäß beschichtbaren implantierbaren medizinischen Vorrichtungen können nahezu beliebige äußere Formen aufweisen; das erfindungsgemäße Verfahren ist nicht auf bestimmte Strukturen beschränkt. Die Implantate können nach dem erfindungsgemäßen Verfahren ganz oder teilweise mit einem Polymerfilm beschichtet werden, der anschließend zu einer kohlenstoffhaltigen Schicht karbonisiert wird.

In bevorzugten Ausführungsformen der vorliegenden Erfindung umfassen die zu beschichtenden medizinischen Implantate Stents, insbesondere Metallstents. Mit dem erfindungsgemäßen Verfahren lassen sich auf ebenso einfache wie vorteilhafte Weise Kohlenstoffbasierte bzw. Kohlenstoffhaltige Oberflächenbeschichtungen auf Stents aus rostfreiem Stahl, Platinhaltigen radiopaken Stahllegierungen, sogenannten PERSS (platinum enhanced radiopaque stainless steel alloys), Kobaltlegierungen, Titanlegierungen, hochschmelzenden Legierungen beispielsweise auf Basis von Niob, Tantal, Wolfram und Molybdän, Edelmetallegierungen, Nitinollegierungen, sowie Magnesiumlegierungen und Mischungen der vorgenannten.

Bevorzugte Implantate im Rahmen der vorliegenden Erfindung sind Stents aus rostfreiem Stahl, insbesondere Fe-18Cr-14Ni-2.5Mo ("316LVM" ASTM F138), Fe-21Cr-10Ni-3.5Mn-2.5Mo (ASTM F 1586), Fe-22Cr-13Ni-5Mn (ASTM F 1314), Fe-23Mn-21Cr-1Mo-1N (Nickelfreier rostfreier Stahl); aus Kobaltlegierungen wie z.B. Co-20Cr-15W-10Ni ("L605" ASTM F90), Co-20Cr-35Ni-10Mo ("MP35N" ASTM F 562), Co-20Cr-16Ni-16Fe-7Mo ("Phynox" ASTM F 1058); Beispiele bevorzugter Titanlegierungen sind CP Titanium (ASTM F 67, Grade 1), Ti-6Al-4V (Alpha/beta ASTM F 136), Ti-6Al-7Nb (alpha/beta ASTM F1295), Ti-15Mo (beta grade ASTM F2066); Stents aus Edelmetalllegierungen, insbesondere Iridiumhaltige Legierungen wie Pt-10Ir; Nitinollegierungen wie martensitische, superelastische und kaltbearbeitete (bevorzugt 40%) Nitinole; sowie Magnesiumlegierungen wie Mg-3A1-1Z.

### POLYMERFILM

Nach dem erfindungsgemäßen Verfahren werden die Implantate mindestens teilweise an einer ihrer äußeren Oberflächen, in bevorzugten Anwendungen an ihrer gesamten äußeren Oberfläche mit einer oder mehreren Polymerfilmlagen beschichtet.

Der Polymerfilm kann in einer Ausführungsform der Erfindung in Form einer Polymerfolie vorliegen, die mittels geeigneter Verfahren, beispielsweise durch Folienschrumpfverfahren, auf das Implantat aufgebracht wird oder auch aufgeklebt werden kann. Thermoplastische Polymerfolien lassen sich auf die meisten Substrate insbesondere auch in erwärmtem Zustand im Wesentlichen festhaftend aufbringen.

Ferner kann der Polymerfilm auch eine Beschichtung des Implantats mit Lacken, polymeren oder teilpolymeren Anstrichen, Tauchbeschichtungen, Sprühbeschichtungen oder Überzügen aus Polymerlösungen oder -suspensionen, sowie auflaminierte Polymerschichten umfassen.

Bevorzugte Überzüge lassen sich durch oberflächliche Parylenierung der Substrate gewinnen. Hierbei werden die Substrate zunächst bei erhöhter Temperatur, üblicherweise etwa 600 °C mit Paracyclophan behandelt, wobei auf den Substraten oberflächlich ein Polymerfilm aus Poly(p-xylylen) ausgebildet wird. Dieser läßt sich in einem nachfolgenden Karbonisierungs- bzw. Pyrolyseschritt in Kohlenstoff umwandeln.

In besonders bevorzugten Ausführungsformen wird die Schrittfolge Parylenierung und Karbonisierung mehrfach wiederholt.

Weitere bevorzugte Ausführungsformen von Polymerfilmen sind Polymerschaumsysteme, beispielsweise Phenolschäume, Polyolefinschäume, Polystyrolschäume, Polyurethanschäume, Fluoropolymerschäume, welche sich in poröse Kohlenstoffschichten umwandeln lassen in einem nachfolgenden Karbonisierungs- bzw. Pyrolyseschritt.

Für die Polymerfilme in Form von Folien, Lacken, polymeren Anstrichen, Tauchbeschichtungen, Sprühbeschichtungen oder Überzügen sowie auflaminierte Polymerschichten können beispielsweise Homo- oder Copolymere von aliphatischen oder aromatischen Polyolefinen wie Polyethylen, Polypropylen, Polybuten, Polyisobuten, Polypenten; Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatine, Hyaluronsäure, Stärke, Cellulosen wie Methylcellulose, Hydroxypropylcellulose, Hydroxypropylmethylcellulose, Carboxymethylcellulose-Phtalat; Wachse, Paraffinwachse, Fischer-Tropsch-Wachse; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phatalat), sowie deren Copolymere, Mischungen und Kombinationen dieser Homo- oder Copolymere, verwendet werden.

Besonders bevorzugt werden geeignete lackbasierte Polymerfilme, beispielsweise aus einem ein- oder zwei-Komponenten-Lack hergestellte Filme bzw. Überzüge, die eine Bindemittelbasis aus Alkydharz, Chlorkautschuk, Epoxidharz, Formaldehydharz, (Meth)Acrylatharz, Phenolharz, Alkylphenolharz, Aminharz, Melaninharz, Ölbasis, Nitrobasis, Vinylesterharz, Novolac^{®} - Epoxidharze, Polyester, Polyurethan, Teer, teerartige Materialien, Teerpech, Bitumen, Stärke, Zellulose, Schellack, Wachse, organische Materialien aus nachwachsenden Rohstoffen oder Kombinationen davon aufweisen.

Besonders bevorzugt sind Lacke auf Basis von Phenol- und/oder Melaminharzen, die gegebenenfalls ganz oder teilweise epoxidiert sein können, z. B. handelsübliche Emballagelacke, sowie Ein- oder Zwei-Komponentenlacke auf Basis gegebenenfalls epoxidierter aromatischer Kohlenwasserstoffharze.

Im erfindungsgemäßen Verfahren können mehrere Schichten aus den genannten Polymerfilmen auf das Implantat aufgebracht werden, welche dann gemeinsam karbonisiert werden. Durch Verwendung unterschiedlicher Polymerfilmmaterialien, evtl. Zusatzstoffe in einzelnen Polymerfilmen oder verschieden dicker Filme können so gezielt Gradientenbeschichtungen auf das Implantat aufgebracht werden, beispielsweise mit veränderlichen Porositäts- oder Adsorptionsprofilen innerhalb der Beschichtungen. Ferner kann die Schrittfolge Polymerfilmbeschichtung und Karbonisierung einmal und ggf. auch mehrfach wiederholt werden um kohlenstoffhaltige Multilayerbeschichtungen auf dem Implantat zu erhalten. Hierbei können die Polymerfilme oder Substrate vorstrukturiert oder mittels Zusatzstoffen modifiziert werden. Auch geeignete Nachbehandlungsschritte wie im weiteren beschrieben können nach jedem oder nach einzelnen Schrittfolgen Polymerfilm-beschichtung und Karbonisierung des erfindungsgemäßen Verfahrens angewendet werden, wie beispielsweise eine oxidative Behandlung einzelner Schichten.

Auch die Verwendung mittels oben genannter Lacke oder Beschichtungslösungen beschichteter Polymerfilme zur Beschichtung der Implantate mittels beispielsweise Kaschiertechniken wie thermisch, Druckgepresst, oder naß-in-naß ist erfindungsgemäß vorteilhaft anwendbar.

In bestimmten Ausführungsformen der vorliegenden Erfindung kann der Polymerfilm mit Zusatzstoffen ausgerüstet sein, welche das Karbonisierungsverhalten des Films und/oder die makroskopischen Eigenschaften der aus dem Verfahren resultierenden kohlenstoffbasierten Substratbeschichtung beeinflussen. Beispiele geeigneter Zusatzstoffe sind Füllstoffe, Porenbildner, Metalle, Metallverbindungen, Legierungen und Metallpulver, Streckmittel, Schmiermittel, Gleitmittel, etc. Beispiele für anorganische Zusatz -oder Füllstoffe sind Siliziumoxide oder Aluminiumoxide, Aluminosilikate, Zeolithe, Zirkonoxide, Titanoxide, Talkum, Graphit, Ruß, Fullerene, Tonmaterialien, Phyllosilikate, Silicide, Nitride, Metallpulver, insbesondere von katalytisch aktiven Übergangsmetallen wie Kupfer, Gold und Silber, Titan, Zirkon, Hafnium, Vanadium, Niob, Tantal, Chrom, Molybdän, Wolfram, Mangan, Rhenium, Eisen, Kobalt, Nickel, Ruthenium, Rhodium, Palladium, Osmium, Iridium oder Platin.

Mittels derartiger Zusatzstoffe im Polymerfilm lassen sich beispielsweise biologische, mechanische und thermische Eigenschaften der Filme wie auch der resultierenden Kohlenstoffbeschichtungen modifizieren und einstellen. So kann z.B. durch den Einbau von Schichtsilikaten, Nanopartikeln, anorganischen Nanokompositen Metallen, Metalloxiden der thermische Ausdehnungskoeffizient der Kohlenstoffschicht dem eines Substrat aus Keramik angeglichen werden, so dass die aufgebrachte kohlenstoffbasierte Beschichtung auch bei starken Temperaturdifferenzen fest haftet. Der Fachmann wird aufgrund einfacher Routineexperimente eine geeignete Kombination aus Polymerfilm und Zusatzstoff auswählen, um für jedes Implantatmaterial die gewünschten Haftungs- und Ausdehnungseigenschaften der kohlenstoffhaltigen Schicht zu erhalten. So wird der Zusatz von Aluminium basierten-Füllstoffen zu einer Erhöhung des thermischen Ausdehnungskoeffizienten und durch den Zusatz von Glas- , Graphit- oder Quartz-basierten Füllstoffen zu einer Verminderung des thermischen Ausdehnungskoeffizienten führen, so dass durch Mischung der Komponenten im Polymersystem der thermische Ausdehnungskoeffizioent entsprechend individuell eingestellt werden kann. Eine weitere mögliche Einstellung der Eigenschaften kann beispielsweise und nicht ausschließlich durch die Herstellung eines Faserverbunds mittels Zusatz von Kohlenstoff-, Polymer-, Glas- oder anderen Fasern in gewebter oder nicht gewebter Form erfolgen, welches zu einer deutlichen Steigerung der Elastizität der Beschichtung führt.

Auch kann die Biokompatibilität der erhaltenen Schichten durch geeignete Wahl von Zusatzstoffen im Polymerfilm verändert und zusätzlich erhöht werden.

In bevorzugten Ausführungsformen der Erfindung lassen sich durch weitere Beschichtung des Polymerfilms mit Epoxydharzen, Phenolharz, Teer, Teerpech, Bitumen, Kautschuk, Polychloropren oder Poly(styrol-co-butadien)-Latex-materialien, Wachse, Siloxane, Silikate, Metallsalze bzw. Metallsalzlösungen, beispielsweise Übergangsmetallsalze, Russ, Fullerene, Aktivkohlepulver, Kohlenstoffmolekularsieb, Perowskit, Aluminiumoxide, Siliziumoxide, Siliziumcarbid, Bornitrid, Siliziumnitrid, Edelmetallpulver wie beispielsweise Pt, Pd, Au oder Ag; sowie Kombinationen davon, oder auch durch gezielten Einbau derartiger Materialien in die Polymerfilmstruktur die Eigenschaften des nach der Pyrolyse und/oder Karbonisierung resultierenden porösen kohlenstoffbasierten Beschichtungen gezielt beeinflussen und veredeln, oder auch MultilayerBeschichtungen herstellen, insbesondere auch Mehrfachbeschichtungen mit Schichten unterschiedlicher Porosität.

Bei der erfindungsgemäßen Herstellung von beschichteten Substraten, besteht durch den Einbau oben genannter Zusatzstoffe in den Polymerfilm die Möglichkeit, die Haftung der aufgebrachten Schicht auf dem Substrat zu verbessern, beispielsweise durch Auftragen von Silanen, Polyanilin oder porösen Titanschichten, und gegebenenfalls den thermischen Ausdehnungskoeffizienten der äußeren Schicht demjenigen des Substrats anzupassen, so dass diese beschichteten Substrate beständiger gegenüber Brüchen in und Abplatzen der Beschichtung werden. Diese Beschichtungen sind somit haltbarer und langzeitstabiler im konkreten Einsatz als herkömmliche Produkte dieser Art.

Die Aufbringung oder der Einbau von Metallen und Metallsalzen, insbesondere auch von Edelmetallen und Übergangsmetallen ermöglicht es, die chemischen, biologischen und adsorptiven Eigenschaften der resultierenden kohlenstoffbasierten Beschichtungen jeweils erwünschten Erfordernissen anzupassen, so dass die resultierende Beschichtung für besondere Anwendungen beispielsweise auch mit heterogenkatalytischen Eigenschaften ausgerüstet werden kann. So können durch Einbau von Silizium-, Titan-, Zirkonium- oder Tantalsalzen während der Karbonisierung die entsprechenden Metallcarbidphasen gebildet werden, die unter Anderem die Oxidationsbeständigkeit der Schicht erhöhen.

Die im erfindungsgemäßen Verfahren verwendeten Polymerfilme haben den Vorteil, dass sie sich einfach in nahezu beliebigen Dimensionen herstellen lassen oder kommerziell erhältlich sind. Polymerfolien und Lacke sind leicht verfügbar, kostengünstig und auf Implantate verschiedenster Art und Form einfach aufzubringen. Die erfindungsgemäß verwendeten Polymerfilme können vor der Pyrolyse bzw. Karbonisierung durch Falten, Prägen, Stanzen, Drucken, Extrudieren, Raffen, Spritzguss und dergleichen in geeigneter Weise strukturiert werden, bevor oder nachdem sie auf das Implantat aufgebracht werden. Auf diese Weise lassen sich in die nach dem erfindungsgemäßen Verfahren hergestellte Kohlenstoffbeschichtung bestimmte Strukturen regelmäßiger oder unregelmäßiger Art einbauen.

### AUFTRAGUNG DES POLYMERFILMS

Die erfindungsgemäß verwendbaren Polymerfilme aus Beschichtungen in Form von Lacken oder Überzügen können aus dem flüssigen, breiigen oder pastenförmigen Zustand, zum Beispiel durch Anstreichen, Streichen, Lackieren, Rakeln, Spincoating, Dispersions- oder Schmelzbeschichten, Extrudieren, Gießen, Tauchen, Sprühen, Drucken oder auch als Hotmelts, aus dem festen Zustand mittels Pulverbeschichtung, Aufsprühen versprühbarer Partikel, Flammspritzverfahren, Sintern oder dergleichen nach an sich bekannten Verfahren auf das Implantat aufbringen. Gegebenenfalls kann das polymere Material hierzu in geeigneten Lösemitteln gelöst oder suspendiert werden. Auch das Kaschieren von geeignet geformten Substraten mit hierfür geeigneten Polymermaterialien oder Folien ist ein erfindungsgemäß verwendbares Verfahren zur Beschichtung des Implantats mit einem Polymerfilm.

Besonders bevorzugt bei der Beschichtung von Stents mit Polymerfilmen ist die Auftragung des Polymers bzw. einer Lösung davon mittels Druckverfahren wie in der DE 10351150 beschrieben, deren Offenbarung hier vollständig mit einbezogen wird. Dieses Verfahren ermöglicht insbesondere eine präzise und reproduzierbare Einstellung der Schichtdicke des aufgetragenen Polymermaterials.

In bevorzugten Ausführungsformen wird der Polymerfilm als flüssiges Polymer oder Polymerlösung in einem geeigneten Lösemittel oder Lösemittelgemisch, ggf. mit anschließender Trocknung, aufgetragen. Geeignete Lösemittel umfassen beispielsweise Methanol, Ethanol, N-Propanol, Isopropanol, Butoxydiglycol, Butoxyethanol, Butoxyisopropanol, Butoxypropanol, n-Butyl-Alkohol, t-Butyl-Alkohol, Butylenglycol, Butyloctanol, Diethylenglycol, Dimethoxydiglycol, Dimethylether, Dipropylenglycol, Ethoxydiglycol, Ethoxyethanol, Ethylhexandiol, Glycol, Hexanediol, 1,2,6-Hexanetriol, Hexylalkohol, Hexylenglycol, Isobutoxypropanol, Isopentyldiol, 3-Methoxybutanol, Methoxydiglycol, Methoxyethanol, Methoxyisopropanol, Methoxymethylbutanol, Methoxy PEG-10, Methylal, Methyl-Hexylether, Methylpropanediol, Neopentylglycol, PEG-4, PEG-6, PEG-7, PEG-8, PEG-9, PEG-6-Methylether, Pentylenglycol, PPG-7, PPG-2-Buteth-3, PPG-2 Butylether, PPG-3 Butylether, PPG-2 Methylether, PPG-3 Methylether, PPG-2 Propylether, Propanediol, Propylenglycol, Propylenglycol-Butylether, Propylenglycol-Propylether, Tetrahydrofuran, Trimethylhexanol, Phenol, Benzol, Toluol, Xylol; als auch Wasser, ggf. im Gemisch mit Dispersionshilfsmitteln, sowie Mischungen der obengenannten davon.

Bevorzugte Lösungsmittel umfassen ein oder mehrere organische Lösungsmittel aus der Gruppe Ethanol, Isopropanol, *n*-Propanol, Dipropylenglykolmethylether und Butoxyisopropanol (1,2-Propylenglykol-*n*-butylether), Tetrahydrofuran, Phenol, Benzol, Toluol, Xylol, vorzugsweise Ethanol, Isopropanol, *n*-Propanol und/oder Dipropylenglykolmethylether, insbesondere Isopropanol und/oder *n*-Propanol.

In bevorzugten Ausführungsformen der vorliegenden Erfindung können die implantierbaren medizinischen Vorrichtungen auch mit mehreren Polymerfilmen aus gleichen Polymeren gleicher oder unterschiedlicher Filmdicke oder verschiedenen Polymeren gleicher oder unterschiedlicher Filmdicke mehrfach beschichtet werden. Auf diese Weise lassen sich beispielsweise tieferliegende porösere Schichten mit darüber liegenden engporigen Schichten kombinieren, welche die Abgabe von in der stärker porösen Schicht deponierten Wirkstoffen geeignet verzögern können.

Alternativ zum Beschichten des Implantats mit einem Polymerfilm und einem nachfolgenden Karbonisierungsschritt ist es erfindungsgemäß auch möglich, auf ein vorerhitztes Implantat ein Polymerfilm-erzeugendes Beschichtungssystem, beispielsweise einen Lack auf Basis von aromatischem Harzen, direkt aufzusprühen, ggf. mit Hilfe von Überdruck, um die aufgesprühte Filmschicht direkt auf der heißen Implantatoberfläche zu karbonisieren.

### KARBONISIERUNG

Der auf das Implantat aufgebrachte Polymerfilm wird gegebenenfalls getrocknet und anschließend einer pyrolytischen Zersetzung unter Karbonisierungsbedingungen unterzogen. Hierbei wird der oder die auf dem Implantat aufbeschichtete(n) Polymerfilm(e) erhitzt, d.h. in einer im Wesentlichen sauerstofffreien Atmosphäre bei erhöhter Temperatur karbonisiert. Die Temperatur des Karbonisierungsschritts liegt vorzugsweise im Bereich von 200°C bis 2500°C und wird vom Fachmann in Abhängigkeit von den spezifischen temperaturabhängigen Eigenschaften der verwendeten Polymerfilme und Implantate gewählt.

Bevorzugte allgemein verwendbare Temperaturen für den Karbonisierungsschritt des erfindungsgemäßen Verfahrens liegen bei 200°C bis etwa 1200°C. Bei einigen Ausführungsformen sind Temperaturen im Bereich von 250°C bis 700°C bevorzugt. Generell wird die Temperatur je nach den Eigenschaften der verwendeten Materialien so gewählt, dass der Polymerfilm mit möglichst geringem Temperaturaufwand im wesentlichen vollständig zu kohlenstoffhaltigem Feststoff überführt wird. Durch die geeignete Wahl bzw. Steuerung der Pyrolysetemperatur kann die Porosität, die Festigkeit und die Steife des Materials sowie weitere Eigenschaften gezielt eingestellt werden.

Abhängig von der Art des verwendeten Polymerfilms und der gewählten Karbonisierungsbedingungen, insbesondere der Atmosphärenzusammensetzung, der gewählten Temperaturen oder Temperaturprogramme und Druckbedingungen kann mit dem erfindungsgemäßen Verfahren die Art und Struktur der abgeschiedenen kohlenstoffhaltigen Schicht gezielt eingestellt bzw. variiert werden. So kommt es beispielsweise bei Verwendung von reinen kohlenwasserstoffbasierten Polymerfilmen in sauerstofffreier Atmosphäre bei Temperaturen bis zu ca. 1000°C zur Abscheidung von im wesentlichen amorphem Kohlenstoff, wohingegen bei Temperaturen oberhalb von 2000°C hochgeordnete kristalline Graphitstrukturen erhalten werden. Im Bereich zwischen diesen beiden Temperaturen lassen sich teilkristalline kohlenstoffhaltige Schichten verschiedener Dichten und Porositäten erhalten.

Ein weiteres Beispiel ist die Verwendung von geschäumten Polymerfilmen, beispielsweise geschäumten Polyurethanen, welche bei Karbonisierung relativ poröse Kohlenstoffschichten mit Porengrößen im unteren Millimeterbereich erhalten lassen. Auch kann durch die Dicke des aufgebrachten Polymerfilms und die gewählten Temperatur- und Druckbedingungen bei der Pyrolyse die Schichtdicke der abgeschiedenen kohlenstoffhaltigen Schicht in weiten Grenzen variiert werden, von Kohlenstoffmonoschichten über nahezu unsichtbare Schichten im Nanometerbereich bis zu Lackschichtstärken von 10 bis 40 Mikrometer Trockenschicht, bis hin zu dickeren Depotschichtstärken im Millimeterbereich bis Zentimeterbereich. Letzteres ist insbesondere bei Implantaten aus Vollkarbonwerkstoffen bevorzugt, insbesondere bei Knochenimplantaten.

Durch geeignete Wahl des Polymerfilm-Materials und der Karbonisierungsbedingungen können so molekularsiebähnliche Depotschichten mit gezielt steuerbaren Porengrößen und Siebeigenschaften erhalten werden, welche die kovalente, adsorbtive oder absorbtive oder auch elektrostatische Anbindung von Wirkstoffen oder Oberflächenmodifikationen ermöglichen.

Vorzugsweise wird Porosität in den erfindungsgemäßen Schichten auf Implantaten durch Behandlungsverfahren erzeugt, wie sie in der DE 103 35 131 und der PCT/EP04/00077 beschrieben werden, deren Offenbarungen hiermit vollständig einbezogen wird.

Die Atmosphäre beim Karbonisierungsschritt des erfindungsgemäßen Verfahrens ist im Wesentlichen frei von Sauerstoff, vorzugsweise mit O₂-Gehalten unter 10 ppm, besonders bevorzugt unter 1 ppm. Bevorzugt ist die Verwendung von Inertgasatmosphären, beispielsweise aus Stickstoff, Edelgasen wie Argon, Neon sowie beliebige andere inerte, nicht mit Kohlenstoff reagierende Gase oder Gasverbindungen sowie auch Mischungen von inerten Gasen. Bevorzugt sind Stickstoff und/oder Argon.

Die Karbonisierung wird üblicherweise bei Normaldruck in Gegenwart von inerten Gasen wie den oben genannten durchgeführt. Gegebenenfalls sind jedoch auch höhere Inertgasdrücke vorteilhaft verwendbar. In einigen Ausführungsformen des erfindungsgemäßen Verfahrens kann die Karbonisierung auch bei Unterdruck bzw. im Vakuum erfolgen.

Der Karbonisierungsschritt wird vorzugsweise in einem diskontinuierlichen Verfahren in geeigneten Öfen stattfinden, kann aber auch in kontinuierlichen Ofenprozessen durchgeführt werden, was gegebenenfalls auch bevorzugt sein kann. Die ggf. strukturierten, vorbehandelten Polymerfilmbeschichteten Implantate werden dabei auf einer Seite dem Ofen zugeführt und am anderen Ende des Ofens wieder austreten. In bevorzugten Ausführungsformen kann das Polymerfilm-beschichtete Implantat im Ofen auf einer Lochplatte, einem Sieb oder dergleichen aufliegen, so dass durch den Polymerfilm während der Pyrolyse bzw. Karbonisierung Unterdruck angelegt werden kann. Dies ermöglicht nicht nur eine einfache Fixierung der Implantate im Ofen, sondern auch eine Absaugung und optimale Durchströmung der Filme bzw. Baugruppen mit Inertgas während der Pyrolyse und/oder Karbonisierung.

Der Ofen kann durch entsprechende Inertgasschleusen in einzelne Segmente unterteilt werden, in welchen nacheinander ein oder mehrere Pyrolyse- bzw. Karbonisierungsschritte, ggf. bei unterschiedlichen Pyrolyse- bzw. Karbonisierungsbedingungen wie zum Beispiel unterschiedlichen Temperaturstufen, unterschiedlichen Inertgasen bzw. Vakuum durchgeführt werden können.

Ferner können in entsprechenden Segmenten des Ofens ggf. auch Nachbehandlungsschritte wie Nachaktivieren durch Reduktion oder Oxidation oder Imprägnierung mit Metallsalzlösungen etc. durchgeführt werden.

Alternativ hierzu kann die Karbonisierung auch in einem geschlossenen Ofen durchgeführt werden, was insbesondere dann bevorzugt ist, wenn die Pyrolyse und/oder Karbonisierung im Vakuum durchgeführt werden soll.

Während der Pyrolyse und/oder Karbonisierung im erfindungsgemäßen Verfahren tritt üblicherweise eine Gewichtsabnahme des Polymerfilms von ca. 5 % bis 95 %, vorzugsweise ca. 40 % bis 90 %, insbesondere 50 % bis 70 %, je nach verwendetem Ausgangsmaterial und Vorbehandlung auf.

Die erfindungsgemäß hergestellte kohlenstoffbasierte Beschichtung auf den Implantaten bzw. Substraten im allgemeinen weist, je nach Ausgangsmaterial, Menge und Art der Füllmaterialien, einen Kohlenstoffgehalt von mindestens 1 Gew.-% auf, vorzugsweise mindestens 25 %, gegebenenfalls auch mindestens 60 % und insbesondere bevorzugt mindestens 75 %. Erfindungsgemäß besonders bevorzugte Beschichtungen weisen einen Kohlenstoffgehalt von mindestens 50 Gew.-% auf.

### NACHBEHANDLUNG

In bevorzugten Ausführungsformen des erfindungsgemäßen Verfahrens werden die physikalischen und chemischen Eigenschaften der kohlenstoffbasierten Beschichtung nach der Pyrolyse bzw. Karbonisierung durch geeignete Nachbehandlungsschritte weiter modifiziert und dem jeweils gewünschten Verwendungszweck angepasst. Geeignete Nachbehandlungen sind beispielsweise reduzierende oder oxidative Nachbehandlungsschritte, bei welchem die Beschichtung mit geeigneten Reduktionsmitteln und/oder Oxidationsmitteln wie Wasserstoff, Kohlendioxid, Stickstoffoxide wie N₂O, Wasserdampf, Sauerstoff, Luft, Salpetersäure und dergleichen sowie ggf. Mischungen dieser behandelt wird.

Die Nachbehandlungsschritte können ggf. bei erhöhter Temperatur, jedoch unterhalb der Pyrolysetemperatur, beispielsweise von 40°C bis 1000°C, vorzugsweise 70°C bis 900°C, besonders bevorzugt 100°C bis 850°C, insbesondere bevorzugt 200°C bis 800°C und insbesondere bei etwa 700 °C durchgeführt werden. In besonders bevorzugten Ausführungsformen wird die erfindungsgemäß hergestellte Beschichtung reduktiv oder oxidativ, oder mit einer Kombination dieser Nachbehandlungsschritte bei Raumtemperatur modifiziert.

Durch oxidative bzw. reduktive Behandlung, oder auch den Einbau von Zusatzstoffen, Füllstoffen oder funktionellen Materialien lassen sich die Oberflächeneigenschaften der erfindungsgemäß hergestellten Beschichtungen gezielt beeinflussen bzw. verändern. Beispielsweise können durch Einbau von anorganischen Nanopartikeln oder Nanokompositen wie Schichtsilikaten die Oberflächeneigenschaften der Beschichtung hydrophilisiert oder hydrophobisiert werden.

Auch können die erfindungsgemäß hergestellten Beschichtungen nachträglich durch Einbau geeigneter Zusatzstoffe mit biokompatiblen Oberflächen ausgestattet und als Arzneistoffträger oder -Depots eingesetzt werden. Hierzu können z.B. Medikamente oder Enzyme in das Material eingebracht werden, wobei erstere ggf. durch geeignete Retardierung und/oder selektive Permeationseigenschaften der Beschichtungen kontrolliert freigesetzt werden können.

Auch kann nach dem erfindungsgemäßen Verfahren die Beschichtung auf dem Implantat geeignet modifiziert werden, z.B. durch Variation der Porengrößen mittels geeigneter oxidativer oder reduktiver Nachbehandlungsschritte, wie Oxidation an Luft bei erhöhter Temperatur, Kochen in oxidierenden Säuren, Laugen, oder Einmischung flüchtiger Bestandteile, die während der Karbonisierung vollständig abgebaut werden und Poren in der kohlenstoffhaltigen Schicht zurücklassen.

Die karbonisierte Beschichtung kann gegebenenfalls auch in einem weiteren optionalen Verfahrensschritt, einem sogenannten CVD-Prozeß (Chemical Vapour Deposition, chemische Gasphasenabscheidung) oder CVI-Prozeß (Chemical Vapour Infiltration) unterzogen werden, um die Oberflächen- oder Porenstruktur und deren Eigenschaften weiter zu modifizieren. Hierzu wird die karbonisierte Beschichtung mit geeigneten, Kohlenstoffabspaltenden Precursorgasen bei hohen Temperaturen behandelt. Auch andere Elemente können damit abgeschieden werden, beispielsweise Silizium. Derartige Verfahren sind im Stand der Technik seit langem bekannt.

Als Kohlenstoff-abspaltende Precursor kommen nahezu alle bekannten gesättigten und ungesättigten Kohlenwasserstoffe mit ausreichender Flüchtigkeit unter CVD-Bedingungen in Frage. Beispiele hierfür sind Methan, Ethan, Ethylen, Acetylen, lineare und verzweigte Alkane, Alkene und Alkine mit Kohlenstoffzahlen von C₁ - C₂₀, aromatische Kohlenwasserstoffe wie Benzol, Naphthalin etc., sowie ein- und mehrfach alkyl-, alkenyl- und alkinylsubstituierte Aromaten wie beispielsweise Toluol, Xylol, Cresol, Styrol, Parylene etc.

Als Keramik-Precursor können BCl₃, NH₃, Silane wie SiH₄, Tetraethoxysilan (TEOS), Dichlorodimethylsilan (DDS), Methyltrichlorosilan (MTS), Trichlorosilyldichloroboran (TDADB), Hexadichloromethylsilyloxid (HDMSO), AlCl₃, TiCl₃ oder Mischungen davon verwendet werden.

Diese Precursor werden in CVD-Verfahren zumeist in geringer Konzentration von etwa 0,5 bis 15 Vol.-% in Mischung mit einem Inertgas, wie beispielsweise Stickstoff, Argon oder dergleichen angewendet. Auch der Zusatz von Wasserstoff zu entsprechenden Abscheidegasgemischen ist möglich. Bei Temperaturen zwischen 500 und 2000°C, vorzugsweise 500 bis 1500°C und besonders bevorzugt 700 bis 1300°C, spalten die genannten Verbindungen Kohlenwasserstofffragmente bzw. Kohlenstoff oder keramische Vorstufen ab, die sich im Porensystem der pyrolysierten Beschichtung im wesentlichen gleichmäßig verteilt niederschlagen, dort die Porenstruktur modifizieren und so zu einer im wesentlichen homogenen Porengröße und Porenverteilung führen.

Mittels CVD-Methoden lassen sich gezielt Poren in der kohlenstoffhaltigen Schicht auf dem Implantat verkleinern, bis hin zur völligen Schließung/Versiegelung der Poren. Hierdurch lassen sich die sorptiven Eigenschaften, wie auch die mechanischen Eigenschaften der Implantatoberfläche maßgeschneidert einstellen.

Durch CVD von Silanen oder Siloxanen, gegebenenfalls im Gemisch mit Kohlenwasserstoffen lassen sich die kohlenstoffhaltigen Implantatbeschichtungen durch Carbid- oder Oxycarbidbildung beispielsweise oxidationsbeständig modifizieren.

In bevorzugten Ausführungsformen können die erfindungsgemäß beschichteten Implantate mittels Sputterverfahren zusätzlich beschichtet bzw. modifiziert werden. Hierzu können Kohlenstoff, Silizium oder Metalle bzw. Metallverbindungen aus geeigneter Sputtertargets nach an sich bekannten Verfahren aufgebracht werden. Beispiele hierfür sind Ti, Zr, Ta, W, Mo, Cr, Cu, die in die kohlenstoffhaltigen Schichten eingestäubt werden können, wobei sich in der Regel die entsprechenden Carbide bilden.

Ferner können mittels Ionenimplantierung die Oberflächeneigenschaften des beschichteten Implantats modifiziert werden. So können durch Implantierung von Stickstoff Nitrid-, Carbonitrid- oder Oxynitridphasen mit eingelagerten Übergangsmetallen gebildet werden, was die chemische Resistenz und mechanische Widerstandsfähigkeit der kohlenstoffhaltigen Implantatbeschichtungen deutlich erhöht. Die Ionenimplantierung von Kohlenstoff kann zur Erhöhung der mechanischen Festigkeit der Beschichtungen wie auch zur Nachverdichtung poröser Schichten verwendet werden.

Ferner ist es in bestimmten Ausfiihrungsformen bevorzugt, die erfindungsgemäß hergestellten Implantatbeschichtungen zu fluoridieren, etwa um Oberflächenbeschichtete Implantate wie z.B. Stents oder Orthopädieimplantate für die Aufnahme lipophiler Wirkstoffe nutzbar zu machen.

In bestimmten Ausführungsformen kann es vorteilhaft sein, die beschichtete implantierbare Vorrichtung mit mindestens einer zusätzlichen Schicht aus biologisch abbaubaren bzw. resorbierbaren Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymere oder nicht-biologisch abbaubaren bzw. resorbierbaren Polymeren zumindest teilweise zu beschichten. Bevorzugt sind insbesondere anionische, kationischen oder amphotere Beschichtungen, wie z.B. Alginat, Carrageenan, Carboxymethylcellulose; Chitosan, Poly-L-Lysine; und/oder Phoshporylcholin.

Sofern erforderlich kann in besonders bevorzugten Ausführungsformen das beschichtete Implantat nach der Karbonisierung und/oder nach gegebenenfalls erfolgten Nachbehandlungsschritten weiteren chemischen oder physikalischen Oberflächenmodifikationen unterzogen werden. Auch Reinigungsschritte zur Entfernung von eventuellen Rückständen und Verunreinigungen können hier vorgesehen werden. Hierzu können Säuren, insbesondere oxidierenden Säuren, oder Lösemittel verwendet werden, bevorzugt ist das Auskochen in Säuren oder Lösemitteln.

Vor der medizinischen Verwendung können die erfindungsgemäß beschichteten Implantate mit üblichen Methoden sterilisiert werden, beispielsweise durch Autoklavieren, Ethylenoxid-Sterilisation oder Gamma-Bestrahlung.

Erfindungsgemäß aus Polymerfilmen erzeugter pyrolytischer Kohlenstoff selbst ist in der Regel ein hoch bioverträgliches Material, das bei medizinischen Anwendungen wie beispielsweise der äußeren Beschichtung von Implantaten verwendet werden kann. Die Biokompatibilität der erfindungsgemäß beschichteten Implantate kann ferner durch den Einbau von Zusatzstoffen, Füllstoffen, Proteinen oder funktionellen Materialien und/oder Medikamente in die Polymerfilme vor oder nach der Karbonisierung gezielt beeinflusst, bzw. verändert werden, wie oben erwähnt. Hierdurch lassen sich Abstoßungsphänomene im Körper bei erfindungsgemäß hergestellten Implantaten verringern oder ganz ausschalten.

In besonders bevorzugten Ausführungsformen können erfindungsgemäß hergestellte kohlenstoffbeschichtete medizinische Implantate durch gezielte Einstellung der Porosität der aufgebrachten Kohlenstoffschicht zur kontrollierten Abgabe von Wirkstoffen aus dem Substrat in die äußere Umgebung verwendet werden. Bevorzugte Beschichtungen sind porös, insbesondere nanoporös. Hierin lassen sich beispielsweise medizinische Implantate, insbesondere auch Stents als Arzneistoffträger mit Depotwirkung verwenden, wobei die kohlenstoffbasierte Beschichtung des Implantats als freisetzungsregulierende Membran genutzt werden kann.

Auch können auf die bioverträglichen Beschichtungen Arzneistoffe aufgebracht werden. Dies ist insbesondere da nützlich, wo Wirkstoffe nicht im oder auf dem Implantat direkt aufgebracht werden können, wie etwa bei Metallen.

Ferner können die erfindungsgemäß hergestellten Beschichtungen in einem weiteren Verfahrensschritt mit Arzneistoffen bzw. Medikamenten beladen werden, oder auch mit Markern, Kontrastmitteln zur Lokalisierung von beschichteten Implantaten im Körper versehen werden, beispielsweise auch mit therapeutischen oder diagnostischen Mengen an radioaktiven Strahlern. Für letzteres sind die erfindungsgemäßen Beschichtungen auf Kohlenstoffbasis besonders geeignet, da sie im Gegensatz zu Polymerschichten von radioaktiver Strahlung nicht verschlechtert bzw. angegriffen werden.

Im medizinischen Bereich erweisen sich die erfindungsgemäß beschichteten Implantate als besonders langzeitstabil, da die kohlenstoffbasierten Beschichtungen neben ihrer hohen Festigkeit auch hinreichend elastisch und flexibel eingestellt werden können, so dass sie den Bewegungen des Implantats, insbesondere bei hochbelasteten Gelenken, folgen können, ohne dass die Gefahr besteht, dass sich Risse bilden oder die Schicht abblättert.

Die Porosität erfindungsgemäß aufgebrachter Beschichtungen auf Implantaten kann insbesondere auch mittels Nachbehandlung mit Oxidationsmitteln, beispielsweise Aktivieren bei erhöhter Temperatur in Sauerstoff oder sauerstoffhaltigen Atmosphären oder Anwendung stark oxidierender Säuren wie konzentrierter Salpetersäure und dergleichen, so angepasst werden, dass die kohlenstoffhaltige Oberfläche auf dem Implantat das Einwachsen von Körpergewebe ermöglicht und fördert. Geeignete Schichten hierzu sind makroporös, mit Porengröße von 0,1 µm bis 1000µm, vorzugsweise 1µm bis 400µm. Die geeignete Porosität kann auch durch eine entsprechende Vorstrukturierung des Implantats oder des Polymerfilms beeinflusst werden. Geeignete Maßnahmen hierzu sind z. B. Prägen, Stanzen, Perforieren, Aufschäumen des Polymerfilms.

### WIRKSTOFFBESCHICHTUNG

In bevorzugten Ausführungsformen können die erfindungsgemäß biokompatibel beschichteten Implantate mit Wirkstoffen, einschließlich Mikroorganismen oder lebenden Zellen, beladen werden. Die Beladung mit Wirkstoffen kann in oder auf der kohlenstoffhaltigen Beschichtung mittels geeigneter sorptiver Methoden wie Adsorption, Absorption, Physisorption, Chemisorption erfolgen, im einfachsten Fall durch Imprägnierung der kohlenstoffhaltigen Beschichtung mit Wirkstofflösungen, Wirkstoffdispersionen oder Wirkstoffsuspensionen in geeigneten Lösungsmitteln. Auch kovalente oder nichtkovalente Anbindung von Wirkstoffen in oder auf der kohlenstoffhaltigen Beschichtung kann hier nach Abhängigkeit des verwendeten Wirkstoffs und seiner chemischen Eigenschaften eine bevorzugte Option sein.

In porösen kohlenstoffhaltigen Beschichtungen können Wirkstoffe in Poren okkludiert werden.

Die Wirkstoffbeladung kann temporär sein, d. h. der Wirkstoff kann nach Implantierung der medizinischen Vorrichtung freigesetzt werden, oder aber der Wirkstoff wird in oder auf der kohlenstoffhaltigen Schicht dauerhaft immobilisiert. Auf diese Weise können wirkstoffhaltige medizinische Implantate mit statischen, dynamischen oder kombiniert statischen und dynamischen Wirkstoffbeladungen erzeugt werden. So ergeben sich multifunktionale Beschichtungen auf Basis der erfindungsgemäß hergestellten kohlenstoffhaltigen Schichten.

Bei statischer Beladung mit Wirkstoffen werden Wirkstoffe im Wesentlichen permanent auf oder in der Beschichtung immobilisiert. Hierfür verwendbare Wirkstoffe sind anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, sowie pharmakologisch wirksame Stoffe oder Stoffgemische, Kombinationen dieser und dergleichen.

Bei dynamischen Wirkstoffbeladungen ist die Freisetzung der aufgebrachten Wirkstoffe nach Implantierung der medizinischen Vorrichtung im Körper vorgesehen. Auf diese Weise können die beschichteten Implantate zu therapeutischen Zwecken eingesetzt werden, wobei die auf das Implantat aufgebrachten Wirkstoffe lokal am Einsatzort des Implantats sukzessive freigesetzt werden. In dynamischen Wirkstoffbeladungen für die Freisetzung von Wirkstoffen verwendbare Wirkstoffe sind beispielsweise Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, und dergleichen, sowie pharmakologisch wirksame Stoffe oder Stoffgemische.

Geeignete pharmakologisch wirksame Stoffe oder Stoffgemische zur statischen und/oder dynamischen Beladung von erfindungsgemäß beschichteten implantierbaren medizinischen Vorrichtungen umfassen Wirkstoffe oder Wirkstoffkombinationen, die ausgewählt sind aus Heparin, synthetische Heparin-Analoga (z.B. Fondaparinux), Hirudin, Antithrombin III, Drotrecogin alpha; Fibrinolytica wie Alteplase, Plasmin, Lysokinasen, Faktor XIIa, Prourokinase, Urokinase, Anistreplase, Streptokinase; Thrombozytenaggregations-hemmer wie Acetylsalicylsäure, Ticlopidine, Clopidogrel, Abciximab, Dextrane; Corticosteroide wie Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone; sogenannte Non-Steroidal Anti-Inflammatory Drugs wie Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib; Zytostatika wie Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purin-oder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, und Antiöstrogene; Antiarrythmika, insbesondere Antiarrhythmika der Klasse I wie Antiarrhythmika vom Chinidintyp, z.B. Chinidin, Dysopyramid, Ajmalin, Prajmaliumbitartrat, Detajmiumbitartrat; Antiarrhythmika vom Lidocaintyp, z.B. Lidocain, Mexiletin, Phenytoin, Tocainid; Antiarrhythmika der Klasse I C, z.B. Propafenon, Flecainid(acetat); Antiarrhythmika der Klasse II, Betarezeptorenblocker wie Metoprolol, Esmolol, Propranolol, Metoprolol, Atenolol, Oxprenolol; Antiarrhythmika der Klasse III wie Amiodaron, Sotalol; Antiarrhythmika der Klasse IV wie Diltiazem, Verapamil, Gallopamil; andere Antiarrhythmika wie Adenosin, Orciprenalin, Ipratropiumbromid; Agenzien zur Stimulation der Angiogenese im Myokard wie Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC); Digitalisglykoside wie Acetyldigoxin/Metildigoxin, Digitoxin, Digoxin; Herzglykoside wie Ouabain, Proscillaridin; Antihypertonika wie zentral wirksame antiadrenerge Substanzen, z.B. Methyldopa, Imidazolinrezeptoragonisten; Kalziumkanalblocker vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker wie Prazosin, Urapidil, Doxazosin, Bunazosin, Terazosin, Indoramin; Vasodilatatoren wie Dihydralazin, Diisopropylamindichloracetat, Minoxidil, Nitroprussidnatrium; andere Antihypertonika wie Indapamid, Co-Dergocrinmesilat, Dihydroergotoxinmethansulfonat, Cicletanin, Bosentan, Fludrocortison; Phosphodiesterasehemmer wie Milrinon, Enoximon und Antihypotonika, wie insbesondere adrenerge und dopaminerge Substanzen wie Dobutamin, Epinephrin, Etilefrin, Norfenefrin, Norepinephrin, Oxilofrin, Dopamin, Midodrin, Pholedrin, Ameziniummetil; und partielle Adrenozeptor-Agonisten wie Dihydroergotamin; Fibronectin, Polylysine, Ethylenevinylacetate, inflammatorische Zytokine wie: TGFβ, PDGF, VEGF, bFGF, TNFα, NGF, GM-CSF, IGF-a, IL-1, IL-8, IL-6, Growth Hormone; sowie adhäsive Substanzen wie Cyanacrylate, Beryllium, Silica; und Wachstumsfaktoren (Growth Factor) wie Erythropoetin, Hormonen wie Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, Octreotid; Bone and Cartilage Stimulating Peptides, bone morphogenetic proteins (BMPs), insbesondere rekombinante BMP's wie z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride wie Dinatriumfluorophosphat, Natriumfluorid; Calcitonin, Dihydrotachystyrol; Growth Factors und Zytokine wie Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs); Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, und Ethanol; ferner Silber(ionen), Titandioxid, Antibiotika und Antiinfektiva wie insbesondere β -Laktam-Antibiotika, z.B. β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine wie Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil; Aztreonam, Ertapenem, Meropenem; β-Lactamase-Inhibitoren wie Sulbactam, Sultamicillintosilat; Tetracycline wie Doxycyclin, Minocyclin, Tetracyclin, Chlortetracyclin, Oxytetracyclin; Aminoglykoside wie Gentamicin, Neomycin, Streptomycin, Tobramycin, Amikacin, Netilmicin, Paromomycin, Framycetin, Spectinomycin; Makrolidantibiotika wie Azithromycin, Clarithromycin, Erythromycin, Roxithromycin, Spiramycin, Josamycin; Lincosamide wie Clindamycin, Lincomycin, Gyrasehemmer wie Fluorochinolone wie Ciprofloxacin, Ofloxacin, Moxifloxacin, Norfloxacin, Gatifloxacin, Enoxacin, Fleroxacin, Levofloxacin; Chinolone wie Pipemidsäure; Sulfonamide, Trimethoprim, Sulfadiazin, Sulfalen; Glykopeptidantibiotika wieVancomycin, Teicoplanin; Polypeptidantibiotika wie Polymyxine wie Colistin, Polymyxin-B, Nitroimidazol-Derivate wie Metronidazol, Tinidazol; Aminochinolone wie Chloroquin, Mefloquin, Hydroxychloroquin; Biguanide wie Proguanil; Chininalkaloide und Diaminopyrimidine wie Pyrimethamin; Amphenicole wie Chloramphenicol; Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid; Virustatika wie Aciclovir, Ganciclovir, Famciclovir, Foscarnet, Inosin-(Dimepranol-4-acetamidobenzoat), Valganciclovir, Valaciclovir, Cidofovir, Brivudin; antiretrovirale Wirkstoffe (nukleosidanaloge Reverse-Transkriptase-Hemmer und -Derivate) wie Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir; Amantadin, Ribavirin, Zanamivir, Oseltamivir und Lamivudin, sowie beliebige Kombinationen und Gemische davon.

### STENTS

Besonders bevorzugte Ausführungsformen der vorliegenden Erfindung, herstellbar nach dem erfindungsgemäßen Verfaren, sind beschichtete Gefäßendoprothesen (intraluminale Endoprothesen) wie Stents, Koronarstents, intravaskulare Stents, periphere Stents und dergleichen.

Diese können mit dem erfindungsgemäßen Verfahren auf einfache Weise biokompatibel beschichtet werden, wodurch beispielsweise die in der perkutanen transluminalen Angioplastie mit herkömmlichen Stents häufig auftretenden Restenosen verhindert werden können.

In bevorzugten Ausführungen der Erfindung kann durch Aktivierung der kohlenstoffhaltigen Beschichtung, beispielsweise mit Luft bei erhöhter Temperatur, die Hydrophilie der Beschichtung erhöht werden, was die Bioverträglichkeit zusätzlich steigert.

In besonders bevorzugten Ausführungsformen werden nach dem erfindungsgemäßen Verfahren mit einer kohlenstoffhaltigen Schicht versehene Stents, insbesondere Koronarstents und periphere Stents, mit pharmakologisch wirksamen Stoffen oder Stoffgemischen beladen. Beispielsweise können die Stentoberflächen für die lokale Unterdrückung von Zelladhäsion, Thrombozytenaggregation, Komplementaktivierung bzw. inflammatorische Gewebereaktionen oder Zellproliferation mit folgenden Wirkstoffen ausgerüstet werden:

Heparin, synthetische Heparin-Analoga (z.B. Fondaparinux), Hirudin, Antithrombin III, Drotrecogin alpha, Fibrinolytica (Alteplase, Plasmin, Lysokinasen, Faktor XIIa, Prourokinase, Urokinase, Anistreplase, Streptokinase), Thrombozytenaggregations-hemmer (Acetylsalicylsäure, Ticlopidine, Clopidogrel, abciximab, Dextrane), Corticosteroide (Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone), sogenannte Non-Steroidal Anti-Inflammatory Drugs (Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib), Zytostatika (Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purin-oder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, Antiöstrogene).

Für systemische, kardiologische Wirkungen können die erfindungsgemäß beschichteten Stents beladen werden mit:

Antiarrythmika, insbesondere Antiarrhythmika der Klasse I (Antiarrhythmika vom Chinidintyp: Chinidin, Dysopyramid, Ajmalin, Prajmaliumbitartrat, Detajmiumbitartrat; Antiarrhythmika vom Lidocaintyp: Lidocain, Mexiletin, Phenytoin, Tocainid; Antiarrhythmika der Klasse I C: Propafenon, Flecainid(acetat)), Antiarrhythmika der Klasse II (Betarezeptorenblocker) (Metoprolol, Esmolol, Propranolol, Metoprolol, Atenolol, Oxprenolol), Antiarrhythmika der Klasse III (Amiodaron, Sotalol), Antiarrhythmika der Klasse IV (Diltiazem, Verapamil, Gallopamil), andere Antiarrhythmika wie Adenosin, Orciprenalin, Ipratropiumbromid; Stimulation der Angiogenese im Myokard: Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC). Weitere Kardiaka sind: Digitalisglykoside (Acetyldigoxin/Metildigoxin, Digitoxin, Digoxin), weitere Herzglykoside (Ouabain, Proscillaridin). Ferner Antihypertonika (zentral wirksame antiadrenerge Substanzen: Methyldopa, Imidazolinrezeptoragonisten; Kalciumkanalblocker: vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker: Prazosin, Urapidil, Doxazosin, Bunazosin, Terazosin, Indoramin; Vasodilatatoren: Dihydralazin, Diisopropylamindichloracetat, Minoxidil, Nitroprussidnatrium), andere Antihypertonika wie Indapamid, Co-Dergocrinmesilat, Dihydroergotoxinmethansulfonat, Cicletanin, Bosentan. Weiters Phosphodiesterasehemmer (Milrinon, Enoximon) und Antihypotonika, hier insbesondere adrenerge und dopaminerge Substanzen (Dobutamin, Epinephrin, Etilefrin, Norfenefrin, Norepinephrin, Oxilofrin, Dopamin, Midodrin, Pholedrin, Ameziniummetil), partielle Adrenozeptor-Agonisten (Dihydroergotamin), schließlich andere Antihypotonika wie Fludrocortison.

Für die Steigerung der Gewebeadhäsion, insbesondere bei peripheren Stents können Komponenten der extrazellulären Matrix, Fibronectin, Polylysine, Ethylenevinylacetate, inflammatorische Zytokine wie: TGFβ, PDGF, VEGF, bFGF, TNFα, NGF, GM-CSF, IGF-a, IL-1, IL-8, IL-6, Growth Hormone; sowie adhäsive Substanzen wie: Cyanoacrylate, Beryllium, oder Silica verwendet werden:

Weitere hierfür geeignete Substanzen, systemisch und/oder lokal wirkend, sind Wachstumsfaktoren (Growth Factor), Erythropoetin.

Auch Hormone können in den Stentbeschichtungen vorgesehen werden, wie beispielsweise Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, und/oder Octreotid.

### ORTHOPÄDISCHE IMPLANTATE

Im Falle von chirurgischen und orthopädischen Implantaten kann es vorteilhaft sein, die Implantate mit einer oder mehreren kohlenstoffhaltigen Schichten auszustatten, die makroporös sind. Geeignete Porengrößen liegen im Bereich von 0,1 bis 1000 µm, bevorzugt bei 1 bis 400 µm, um eine bessere Integration der Implantate durch Einwachsen ins umliegende Zell- oder Knochengewebe zu unterstützen.

Für orthopädische und nichtorthopädische Implantate sowie erfindungsgemäß beschichtete Herzklappen oder Kunstherzteile können ferner, sofern diese mit Wirkstoffen beladen werden sollen, für die lokale Unterdrückung von Zelladhäsion, Thrombozytenaggregation, Komplementaktivierung bzw. inflammatorische Gewebereaktion oder Zellproliferation die gleichen Wirkstoffe eingesetzt werden wie in den oben beschriebenen Stentanwendungen.

Ferner können zur Stimulation von Gewebewachstum insbesondere bei orthopädischen Implantaten für eine bessere Implantatintegration folgende Wirkstoffe verwendet werden: Bone and Cartilage Stimulating Peptides, bone morphogenetic proteins (BMPs), insbesondere rekombinante BMP's (z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride (Dinatriumfluorophosphat, Natriumfluorid); Calcitonin, Dihydrotachystyrol. Ferner alle Wachstumsfaktoren und Zytokine wie Epidermal Growth Factor (EGF), Platelet-Derived Growth Factor (PDGF), Fibroblast Growth Factors (FGFs), Transforming Growth Factors-b TGFs-b, Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs)). Weitere adhäsions- und integrationsfördernde Substanzen sind neben den bereits genannten inflammatorischen Cytokinen das Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, Ethanol.

### BESONDERE AUSFÜHRUNGSFORMEN

Darüber hinaus können die erfindungsgemäß beschichteten Implantate, insbesondere Stents und dergleichen auch anstelle von Pharmazeutika oder zusätzlich mit antibakteriellen-antiinfektiösen Beschichtungen versehen werden, wobei die folgenden Stoffe oder Stoffgemische verwendbar sind: Silber(ionen), Titandioxid, Antibiotika und Antiinfektiva. Insbesondere beta-Laktam-Antibiotika (β-Lactam-Antibiotika: β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine (Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil) oder andere wie Aztreonam, Ertapenem, Meropenem. Weitere Antibiotika sind β-Lactamase-Inhibitoren (Sulbactam, Sultamicillintosilat), Tetracycline (Doxycyclin, Minocyclin, Tetracyclin, Chlortetracyclin, Oxytetracyclin), Aminoglykoside (Gentamicin, Neomycin, Streptomycin, Tobramycin, Amikacin, Netilmicin, Paromomycin, Framycetin, Spectinomycin), Makrolidantibiotika (Azithromycin, Clarithromycin, Erythromycin, Roxithromycin, Spiramycin, Josamycin), Lincosamide (Clindamycin, Lincomycin), Gyrasehemmer (Fluorochinolone wie Ciprofloxacin, Ofloxacin, Moxifloxacin, Norfloxacin, Gatifloxacin, Enoxacin, Fleroxacin, Levofloxacin; andere Chinolone wie Pipemidsäure), Sulfonamide und Trimethoprim (Sulfadiazin, Sulfalen, Trimethoprim), Glykopeptidantibiotika (Vancomycin, Teicoplanin), Polypeptidantibiotika ( Polymyxine wie Colistin, Polymyxin-B), Nitroimidazol-Derivate ( Metronidazol, Tinidazol), Aminochinolone (Chloroquin, Mefloquin, Hydroxychloroquin), Biguanide (Proguanil), Chininalkaloide und Diaminopyrimidine (Pyrimethamin), Amphenicole (Chloramphenicol) und andere Antibiotika (Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid). Unter den Virustatika sind zu nennen Aciclovir, Ganciclovir, Famciclovir, Foscarnet, Inosin-(Dimepranol-4-acetamidobenzoat), Valganciclovir, Valaciclovir, Cidofovir, Brivudin. Dazu zählen auch Antiretrovirale Wirkstoffe (nukleosidanaloge Reverse-Transkriptase-Hemmer und -Derivate: Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir) und andere Virustatika wie Amantadin, Ribavirin, Zanamivir, Oseltamivir, Lamivudin.

In besonders bevorzugten Ausführungsformen der vorliegenden Erfindung können die erfindungsgemäß hergestellten kohlenstoffhaltigen Schichten vor oder nach der Wirkstoffbeladung mittels weiterer Agenzien in ihren chemischen oder physikalischen Eigenschaften geeignet modifiziert werden, beispielsweise um die Hydrophilie, Hydrophobie, elektrische Leitfähigkeit, Haftung oder sonstige Oberflächeneigenschaften zu modifizieren. Hierfür einsetzbare Stoffe sind biodegradierbare oder nicht-degradierbare Polymere, wie beispielsweise bei den biodegradierbaren: Kollagene, Albumin, Gelatin, Hyaluronsäure, Stärke, Cellulose (Methylcellulose, Hydroxypropylcellulose, Hydroxypropylmethylcellulose, Carboxymethylcellulose-Phtalat; weiterhin Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvaleric Acid), Polydioxanone, Poly(Ethylen-Terephtalate), Poly(malatsäure), Poly(Tartronsäure), Polyanhydride, Polyphosphohazene, Poly(Aminosäuren), und alle ihre Co-Polymere.

Zu den nicht-biodegradierbaren zählen: Poly(Ethylen-Vinylacetate), Silicone, Acrylpolymere wie Polyacrylsäure, Polymethylacrylsäure, Polyacrylcynoacrylat; Polyethylene, Polypropylene, Polyamide, Polyurethane, Poly(Ester-Urethane), Poly(Ether-Urethane), Poly(Ester-Harnstoffe), Polyether wie Polyethylenoxid, Polypropylenoxid, Pluronics, PolyTetramethylenglycol; Vinylpolymere wie Polyvinylpyrrolidone, Poly(Vinyl-alkohole), Poly(vinyl-acetat-phatalat.

Generell gilt, dass Polymere mit anionischen (z.B. Alginat, Carrageenan, carboxymethylcellulose) oder kationischen (z.B. Chitosan, Poly-L-Lysine etc.) oder beiden Eigenschaften (Phoshporylcholin) hergestellt werden können.

Zur Modifizierung der Freisetzungseigenschaften wirkstoffhaltiger erfindungsgemäß beschichteter Implantate können durch Auftragen beispielsweise von weiteren Polymeren spezifische pH- oder temperaturabhängige Freisetzungseigenschaften erzeugt werden. PH-sensitive Polymere sind beispielsweise Poly(Acrylsäure) und Derivate, zum Beispiel: Homopolymere wie Poly(Aminocarboxylsäure), Poly(Acrylsäure), Poly(Methyl-Acrylsäure) und deren Co-Polymere. Ebenso gilt dies für Polysaccharide wie Celluloseacetat-Phtalat, Hydroxypropylmethylcellulose-Phtalat, Hydroxypropylmethylcellulose-Succinat, Celluloseacetat-Trimellitat und Chitosan. Thermosensitive Polymere sind beispielsweise Poly(N-Isopropylacrylamid-Co-Natrium-Acrylat-Co-n-N-Alkylacrylamid), Poly(N-Methyl-N-n-propylacrylamid), Poly(N-Methyl-N-Isopropylacrylamid), Poly(N-n-Propylmethacrylamid), Poly(N-Isopropylacrylamid), Poly(N,n-Diethylacrylamid), Poly(N-Isopropylmethacrylamid), Poly(N-Cyclopropylacrylamid), Poly(N-Ethylacrylamid), Poly(N-Ethylmethyacrylamid), Poly(N-Methyl-N-Ethylacrylamid), Poly(N-Cyclopropylacrylamid). Weitere Polymere mit Thermogel-Charakteristik sind Hydroxypropyl-Cellulose, Methyl-Cellulose, Hydroxypropylmethyl-Cellulose, Ethylhydroxyethyl-Cellulose und Pluronics wie F-127, L-122, L-92, L-81, L-61.

Die Wirkstoffe können einerseits in den Poren der kohlenstoffhaltigen Schicht adsorbiert werden (nicht-kovalent, kovalent), wobei deren Freisetzung primär durch Porengröße und -geometrie steuerbar sind. Zusätzliche Modifikationen der porösen Kohlenstoffschicht durch chemische Modifikation (anionisch, kationisch) erlauben die Freisetzung zu modifizieren, beispielsweise pH-abhängig. Eine weitere Anwendung stellt die Freisetzung von wirkstoffhaltigen Trägern dar, nämlich Microcapsules, Liposomen, Nanocapsules, Nanopartikeln, Micellen, synthetische Phospholipide, Gas-Dispersionen, Emulsionen, Mikroemulsionen, Nanospheres etc., die in den Poren der Kohlenstoffschicht adsorbiert und dann therapeutisch freigesetzt werden. Durch zusätzliche kovalente oder nicht-kovalente Modifikation der Kohlenstoffschicht lassen sich die Poren okkludieren, so dass biologisch aktive Wirkstoffe geschützt sind. In Frage kommen die oben bereits genannten Polysaccharide, Lipide etc., allerdings auch die genannte Polymere.

Bei der zusätzlichen Beschichtung der erfindungsgemäß erzeugten porösen kohlenstoffhaltigen Schichten kann daher zwischen physischen Barrieren wie inerten biodegradierbaren Substanzen (z.B. Poly-1-Lysin, Fibronectin, Chitosan, Heparin etc.) und biologisch aktiven Barrieren unterschieden werden. Letztere können sterisch behindernde Moleküle sein, die physiologisch bioaktiviert werden und die Freisetzung von Wirkstoffen bzw. deren Trägern gestatten. Beispielsweise Enzyme, welche die Freisetzung vermitteln, biologisch aktive Stoffe aktivieren oder nichtaktive Beschichtungen binden und zur Exposition von Wirkstoffen führen. Alle hier im speziellen aufgeführten Mechanismen und Eigenschaften sind sowohl auf die primäre erfindungsgemäß erzeugte Kohlenstoffschicht anzuwenden, als auch auf darauf zusätzlich aufgebrachte Schichten.

Die erfindungsgemäß beschichteten Implantate können in besonderen Anwendungen auch mit lebenden Zellen oder Mikroorganismen beladen werden. Diese können sich in geeignet porösen kohlenstoffhaltigen Schichten ansiedeln, wobei das so besiedelte Implantat dann mit einem geeigneten Membranüberzug versehen werden kann, der für Nährstoffe und von den Zellen oder Mikroorganismen erzeugte Wirkstoffe durchlässig ist, nicht jedoch für die Zellen selbst.

Auf diese Weise lassen sich unter Anwendung der erfindungsgemäßen Technologie beispielsweise Implantate herstellen, die insulinproduzierende Zellen enthalten, welche nach Implantierung im Körper in Abhängigkeit vom Glukosespiegel der Umgebung Insulin produzieren und freisetzen.

### BEISPIELE

Die folgenden Beispiele dienen der Veranschaulichung der erfindungsgemäßen Prinzipien und sind nicht einschränkend gedacht. Im Einzelnen werden verschiedene Implantate oder Implantatmaterialien nach dem erfindungsgemäßen Verfahren beschichtet und deren Eigenschaften, insbesondere hinsichtlich der Biokompatibilität, bestimmt.

### Beispiel 1: Kohlenstoff

Ein erfindungsgemäß beschichtetes Kohlenstoffmaterial wurde wie folgt hergestellt: Auf ein Papier mit 38g/m² Flächengewicht wurde ein Polymerfilm aufgetragen, indem das Papier mehrfach mit einer Rakel mit einem handelsüblichen epoxidierten Phenolharzlack beschichtet und bei Raumtemperatur getrocknet wurde. Trockengewicht 125g/m². Die Pyrolyse bei 800°C über 48 Stunden unter Stickstoff liefert bei einem Schrumpf von 20% und einen Gewichtsverlust von 57% ein asymmetrisch aufgebautes Kohlenstoffblatt mit folgenden Abmessungen: Gesamtstärke 50 Mikrometer, mit 10 Mikrometern einer dichten erfindungsgemäßen kohlenstoffhaltigen Schicht auf einem offenporiger Kohlenstoffträger mit einer Stärke von 40 Mikrometern, der sich unter den Pyrolysebedingungen in situ aus dem Papier bildete. Das Absorptionsvermögen des beschichteten Kohlenstoffmaterials betrug bis zu 18 g Ethanol / m².

### Beispiel 2: Glas

Duroplan® Glas wird einer 15 min. Ultraschallreinigung in einem tensidhaltigen Wasserbad unterworfen, mit destilliertem Wasser und Aceton gespült und getrocknet. Dieses Material wird durch Tauchbeschichtung mit einem handelsüblichen Emballagelack auf Phenoharzbasis mit 2,0*10⁻⁴ g/cm² Auftragsgewicht beschichtet Nach anschließender Karbonisierung bei 800°C über 48 Stunden unter Stickstoff tritt ein Gewichtsverlust der Beschichtung auf 0,33 * 10⁻⁴ g/cm² ein. Die zuvor farblose Beschichtung wird schwarz glänzend, und ist nach der Karbonisierung kaum mehr durchsichtig. Ein Test der Beschichtungshärte mit einem Bleistift, der in einem Winkel von 45° mit einem Gewicht von 1kg über die beschichtete Fläche gezogen wird ergibt bis zu einer Härte von 5H keine optisch wahrnehmbare Beschädigung der Oberfläche.

### Beispiel 3: Glas, CVD-Beschichtung (Vergleichsbeispiel)

Duroplan® Glas wird einer 15 min. Ultraschallreinigung unterworfen, mit dest. Wasser und Aceton gespült und getrocknet. Dieses Material wird durch chemische Dampfabscheidung (CVD) mit 0,05*10⁻⁴ g/cm² Kohlenstoff beschichtet Hierzu wird Benzol bei 30°C in einem Blubberer durch einen Stickstofffluß über 30 Minuten in Kontakt mit der 1000°C heißen Glasoberfläche gebracht und als Film auf der Glasoberfläche abgeschieden. Die zuvor farblose Glasoberfläche wird grau glänzend und ist nach der Abscheidung gedämpft durchsichtig. Ein Test der Beschichtungshärte mit einem Bleistift, der in einem Winkel von 45° mit einem Gewicht von 1kg über die beschichtete Fläche gezogen wird ergibt bis zu einer Härte von 6B keine optisch wahrnehmbare Beschädigung der Oberfläche.

### Beispiel 4: Glasfaser

Duroplan® Glasfasern mit 200 Mikrometern Durchmesser, werden einer 15 min. Ultraschallreinigung unterworfen, mit dest. Wasser und Aceton gespült und getrocknet. Dieses Material wird durch Tauchbeschichtung mit einem handelsüblichen Emballagelack mit 2,0* 10⁻⁴ g/cm² Auftragsgewicht beschichtet. Nach anschließender Pyrolyse mit Karbonisierung bei 800°C über 48 Stunden tritt ein Gewichtsverlust der Beschichtung auf 0,33* 10⁻⁴ g/cm² ein. Die zuvor farblose Beschichtung wird schwarz glänzend, und ist nach der Karbonisierung kaum mehr durchsichtig. Ein Test der Haftung der Beschichtung durch Biegung im Radius von 180° ergibt keine Abplatzungen, d.h. optisch wahrnehmbare Beschädigung der Oberfläche.

### Beispiel 5: Edelstahl

Edelstahl 1.4301 als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,0* 10⁻⁴ g/cm² Auftragsgewicht beschichtet. Nach anschließender Pyrolyse mit Karbonisierung bei 800°C über 48 Stunden unter Stickstoff tritt ein Gewichtsverlust der Beschichtung auf 0,49* 10⁻⁴ g/cm² ein. Die zuvor farblose Beschichtung wird nach der Karbonisierung matt schwarz. Ein Test der Beschichtungshärte mit einem Bleistift, der in einem Winkel von 45° mit einem Gewicht von 1kg über die beschichtete Fläche gezogen wird ergibt bis zu einer Härte von 4B keine optisch wahrnehmbare Beschädigung der Oberfläche.
Ein Klebestreifen-Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt kaum Anhaftungen.

### Beispiel 6: Edelstahl, CVD-Beschichtung (Vergleichsbeispiel)

Edelstahl 1.4301 als 0,1mm Folie (Goodfellow) wird einer 15 min. Ultraschallreinigung unterworfen, mit dest. Wasser und Aceton gespült und getrocknet. Dieses Material wird durch chemische Dampfabscheidung (CVD) mit 0,20*10⁻⁴ g/cm² beschichtet. Hierzu wird Benzol bei 30°C in einem Blubberer durch einen Stickstofffluss über 30 Minuten in Kontakt mit der 1000°C heißen Metalloberfläche gebracht, bei den hohen Temperaturen zersetzt und als Film auf der Metalloberfläche abgeschieden. Die zuvor metallische Oberfläche wird schwarz glänzend nach der Abscheidung. Ein Test der Beschichtungshärte mit einem Bleistift, der in einem Winkel von 45° mit einem Gewicht von 1kg über die beschichtete Fläche gezogen wird ergibt bis zu einer Härte von 4B keine optisch wahrnehmbare Beschädigung der Oberfläche.
Ein Tesafilm Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt deutlich sichtbare, graue Anhaftungen.

### Beispiel 7: Titan

Titan 99,6% als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,2*10⁻⁴ g/cm² beschichtet. Nach anschließender Pyrolyse mit Karbonisierung bei 800°C über 48 Stunden unter Stickstoff tritt ein Gewichtsverlust der Beschichtung auf 0,73*10⁻⁴ g/cm² ein. Die zuvor farblose Beschichtung wird matt, grau-schwarz glänzend. Ein Test der Beschichtungshärte mit einem Bleistift, der in einem Winkel von 45° mit einem Gewicht von 1kg über die beschichtete Fläche gezogen wird ergibt bis zu einer Härte von 8H keine optische Beschädigung der Oberfläche. Auch beispielweise mit einer Büroklammer kann die Beschichtung nicht zerkratzt werden. Ein Abziehtest, bei dem ein Tesa®-Streifen mit mindestens 3 cm Länge über 60 Sekunden mit dem Daumen auf die Oberfläche geklebt wird und anschließend im Winkel von 90° von der Oberfläche wieder abgezogen wird, ergibt keine Anhaftungen.

### Beispiel 8: Titan, veredelt mit CVD

Titan 99,6% als 0,1mm Folie (Goodfellow) wird einer 15 min. Ultraschallreinigung unterworfen, mit dest. Wasser und Aceton gespült und getrocknet. Dieses Material wird durch Tauchbeschichtung mit einem handelsüblichen Emballagelack mit 2,2* 10⁻⁴ g/cm² beschichtet. Nach anschließender Pyrolyse mit Karbonisierung bei 800°C über 48 Stunden unter Stickstoff tritt ein Gewichtsverlust der Beschichtung auf 0,73 * 10⁻⁴ g/cm² ein. Dieses Material wird durch chemische Dampfabscheidung (CVD) weiter beschichtet mit 0,10*10⁻⁴ g/cm². Hierzu wird Benzol bei 30°C in einem Blubberer durch einen Stickstofffluss über 30 Minuten in Kontakt mit der 1000°C heißen, beschichteten Metalloberfläche gebracht, zersetzt und als Film auf der Oberfläche abgeschieden. Die zuvor metallische Oberfläche wird schwarz glänzend nach der Abscheidung. Nach der Abkühlung auf 400°C wird die Oberfläche durch Überleiten von Luft für die Dauer von 3 Stunden oxidiert. Ein Test der Beschichtungshärte mit einem Bleistift, der in einem Winkel von 45° mit einem Gewicht von 1kg über die beschichtete Fläche gezogen wird ergibt bis zu einer Härte von 8H keine optisch wahrnehmbare Beschädigung der Oberfläche.
Ein Abziehtest, bei dem ein Tesa®-Klebebandstreifen mit mindestens 3 cm Länge über 60 Sekunden mit dem Daumen auf die Oberfläche geklebt wird und anschließend im Winkel von 90° von der Oberfläche wieder abgezogen wird, ergibt graue Anhaftungen.

### Beispiel 9

Die Titan Oberflächen werden am in-vitro Petrischalen-Modell hinsichtlich ihrer Biokompatibilität mittels üblicher Testverfahren getestet. Dabei werden 16 cm² große Stücke aus den beschichteten Materialien der Beispiele 2, 7 und 8 ausgestanzt und mit Blut bei 37°C, 5% CO₂ für 3h inkubiert. Zum Vergleich wurden gleich große Flächen der unbeschichteten Materialien Titan und Glas untersucht. Die Versuche werden mit n=3 Spendern durchgeführt und pro Oberfläche mit drei Probekörpern vermessen. Die Proben werden entsprechend vorbereitet und die verschiedenen Parameter (Blutplättchen, TAT (Thrombin-Antithrombin-Komplex)- und C5a-Aktivierung) bestimmt.

Die Messwerte werden gegen einen Leerwert als Kontrolle, entsprechend einer nahezu idealen, extrem optimierten Biokompatibilität, und zwei kommerziell erhältliche Dialysemembranen (Cuprophan® und Hemophan®) getestet, um einen Vergleichsmaßstab zu erhalten. Die Ergebnisse sind in Tabelle I zusammengefasst.

**Tabelle I: Biokompatibilitätstest**

| **Material** | **Blutplättchen zahl (%)** | **TAT (ng/ml)** | **C5a (ng/ml)** |
|---|---|---|---|
| 1. Leerwert | 86,6 | 3,1 | 3,1 |
| 2. Cuprophan Rolle 05/3126-42 | 64,8 | 40,2 | 70,4 |
| 3. Hemophan Typ 80 MC 81-512461 | 71,0 | 32,9 | 29,8 |
| 4. Titan 99,6%, beschichtet, aus Beispiel 7 | 73,3 | 194,3 | 3,9 |
| 5. Titan 99,6%, veredelt, aus Beispiel 8 | 67,0 | 11,1 | 10,8 |
| 6. Titan 99,6%, Kontrolle | 59,6 | >1200,0 | 14,5 |
| 7. Duroplan Glas, beschichtet, aus Beispiel 2 | 73,7 | 137,1 | 11,4 |
| 8. Duroplan Glas Kontrolle | 49,1 | >1233,3 | 25,5 |

Die Ergebnisse zeigen eine zum Teil deutliche Verbesserung der Biokompatibilität der erfindungsgemäßen Beispiele, sowohl gegenüber den Dialysemembranen als auch gegenüber den unbeschichteten Proben.

### Beispiel 10: Zellwachstumstest

Die beschichtete Titan-Oberfläche aus Beispiel 8 und der amorphe Kohlenstoff aus Beispiel 1 wurden weiterhin auf Zellwachstum von Mäuse-L929-Fibroplasten untersucht. Als Vergleich dient eine unbeschichtete Titanoberfläche. Hierzu wurden auf die zuvor dampfsterilisierten Proben 3x10⁴ Zellen pro Probekörper appliziert und über 4 Tage bei optimalen Bedingungen bebrütet. Anschließend wurden die Zellen geerntet und die Zahl pro 4 ml Medium automatisch bestimmt. Jede Probe wurde doppelt vermessen und der Mittelwert gebildet. Die Ergebnisse sind in Tabelle II angegeben:

**Tabelle II: Zellwachstum auf beschichtetem Titan**

| Probematerial | Zellenzahl pro 4 ml |
|---|---|
| Kohlenstoff nach Beispiel 1 | 6,6 |
| Titan 99,6%, Kontrolle | 4,9 |
| Titan, veredelt, aus Beispiel 8 | 7,8 |

Dieses Experiment zeigt eindrucksvoll die Biokompatibilität und Zellwachstumsfördernde Wirkung der erfindungsgemäß beschichteten Oberflächen, insbesondere bei Vergleich der beiden Titanoberflächen.

### Beispiel 11: Beschichteter Stent

Ein kommerziell erhältlicher Metall-Stent der Fa. Baun Melsungen AG, Typ Coroflex 2,5x19mm wird einer 15 min. Ultraschallreinigung im tensidhaltigen Wasserbad unterworfen, mit destilliertem Wasser und Aceton gespült und getrocknet. Dieses Material wird durch Tauchbeschichtung mit einem handelsüblichen Emballagelack auf Phenolharz/Melaminharzbasis mit 2,0*10⁻⁴ g/cm² beschichtet. Nach anschließender Pyrolyse mit Karbonisierung bei 800°C über 48 Stunden unter Stickstoff tritt ein Gewichtsverlust der Beschichtung auf 0,49* 10⁻⁴ g/cm² ein. Die zuvor metallisch hochglänzende Oberfläche wird dabei matt schwarz. Für einen Test der Haftung der Beschichtung durch Expansion des Stents mit 6 bar auf die Nenngröße von 2,5 mm wurde der beschichtete Stent mit einem Ballonkatheter aufgedehnt. Die anschließende optische Begutachtung mit einer Lupe ergab mikroskopisch keine Abplatzungen der homogenen Beschichtung von der Metalloberfläche. Das Absorptionsvermögen dieser porösen Schicht betrug bis zu 0,005 g Ethanol.

### Beispiel 12: Beschichteter Carbostent

Ein kommerziell erhältlicher kohlenstoffbeschichteter Metallstent der Fa. Sorin Biomedica, Typ Radix Carbostent 5x12mm wird einer 15 min. Ultraschallreinigung unterworfen, mit destilliertem Wasser und Aceton gespült und getrocknet. Dieses Material wird durch Tauchbeschichtung mit einem handelsüblichen Emballagelack auf Phenolharz/Melaminharz-basis mit 2,0*10⁻⁴ g/cm² Auftragsgewicht beschichtet. Nach anschließender Pyrolyse mit Karbonisierung bei 800°C über 48 Stunden unter Stickstoff tritt ein Gewichtsverlust der Beschichtung auf 0,49* 10⁻⁴ g/cm² ein. Die zuvor schwarze Oberfläche wird nach der Karbonisierung matt schwarz. Für einen Test der Haftung der Beschichtung durch Expansion des Stents mit 6 bar auf die Nenngröße von 5 mm wurde der beschichtete Stent aufgedehnt. Die anschließende optische Begutachtung mit einer Lupe ergab mikroskopisch keine Abplatzungen der homogenen Beschichtung von der Metalloberfläche. Das Absorptionsvermögen dieser porösen Schicht des o.g. Stentmodells betrug bis zu 0,005 g Ethanol.

### Beispiel 13: Aktivierung

Der beschichtete Stent aus Beispiel 12 wird durch Aktivierung mit Luft bei 400°C über 8 Stunden aktiviert. Hierbei wird die Kohlenstoffbeschichtung in porösen Kohlenstoff überführt. Für einen Test der Haftung der Beschichtung durch Expansion des Stents mit 6 bar auf die Nenngröße von 5 mm wurde der beschichtete Stent aufgedehnt. Die anschließende optische Begutachtung mit einer Lupe ergab mikroskopisch keine Abplatzungen der homogenen Beschichtung von der Metalloberfläche. Das Absorptionsvermögen dieser nun porösen Schicht des o.g. Stentmodells betrug bis zu 0,007 g Ethanol, was zeigt, dass eine zusätzliche Aktivierung der kohlenstoffhaltigen Schicht das Aufnahmevermögen zusätzlich erhöht.

Die Anmeldung beinhaltet darüber hinaus die folgenden Ausführungsformen:
1. Verfahren zur Herstellung biokompatibler Beschichtungen auf implantierbaren, medizinischen Vorrichtungen, umfassend die folgenden Schritte:
   a) mindestens partielles Beschichten der medizinischen Vorrichtung mit einem Polymerfilm mittels eines geeigneten Beschichtungs- bzw. Auftragungsverfahrens,
   b) Erhitzen des Polymerfilms in einer Atmosphäre, die im Wesentlichen frei von Sauerstoff ist, auf Temperaturen im Bereich von 200°C bis 2500°C,
   zur Erzeugung einer kohlenstoffhaltigen Schicht auf der medizinischen Vorrichtung.
2. Verfahren nach Ausführungsform 1,
   **dadurch gekennzeichnet,** dass die implantierbare medizinische Vorrichtung aus einem Material besteht, welches aus Kohlenstoff, Kohlenstoffverbundmaterial, Kohlefasern, Keramik, Glas, Metalle, Legierungen, Knochen, Stein, Mineralien oder Vorstufen dieser oder aus Materialien ausgewählt ist, die unter Karbonisierungsbedingungen in ihren thermostabilen Zustand überführt werden.
3. Verfahren nach einer der vorhergehenden Ausführungsformen,
   **dadurch gekennzeichnet,** dass die implantierbare, medizinische Vorrichtung ausgewählt ist aus medizinischen oder therapeutischen Implantaten wie Gefäßendoprothesen, Stents, Koronarstents, peripheren Stents, orthopädischen Implantaten, Knochen- oder Gelenkprothesen, Kunstherzen, künstliche Herzklappen, subkutane und/oder intramuskuläre Implantate und dergleichen.
4. Verfahren nach einer der vorhergehenden Ausführungsformen,
   **dadurch gekennzeichnet,** dass der Polymerfilm umfasst: Homo- oder Copolymere von aliphatischen oder aromatischen Polyolefinen wie Polyethylen, Polypropylen, Polybuten, Polyisobuten, Polypenten; Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Wachse, Paraffinwachse, Fischer-Tropsch-Wachse; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phatalat), sowie deren Copolymere, Mischungen und Kombinationen dieser Homo- oder Copolymere.
5. Verfahren nach einer der Ausführungsformen 1 bis 3,
   **dadurch gekennzeichnet,** dass der Polymerfilm Alkydharz, Chlorkautschuk, Epoxidharz, Acrylatharz, Phenolharz, Aminharz, Melaminharz, Alkylphenolharze, epoxidierte aromatische Harze, Ölbasis, Nitrobasis, Polyester, Polyurethan, Teer, teerartige Materialien, Teerpech, Bitumen, Stärke, Zellulose, Wachse, Schellack, organische Materialien aus nachwachsenden Rohstoffen oder Kombinationen davon umfasst.
6. Verfahren nach einer der vorhergehenden Ausführungsformen,
   **dadurch gekennzeichnet,** dass der Polymerfilm als flüssiges Polymer oder Polymerlösung in einem geeigneten Lösemittel oder Lösemittelgemisch, ggf. mit anschließender Trocknung, oder als Polymerfeststoff, ggf. in Form von Folien oder versprühbaren Partikeln aufgetragen wird.
7. Verfahren nach Ausführungsform 6,
   **dadurch gekennzeichnet,** dass der Polymerfilm durch Auflaminieren, Kaschieren, Kleben, Tauchen, Sprühen, Drucken, Rakeln, Spincoating, Pulverbeschichtung, oder Flammspritzen auf die Vorrichtung aufgebracht wird.
8. Verfahren nach einer der vorhergehenden Ausführungsformen,
   ferner umfassend den Schritt des Abscheidens von Kohlenstoff und/oder Silizium mittels chemischer oder physikalischer Dampfphasenabscheidung (CVD bzw. PVD).
9. Verfahren nach einer der vorhergehenden Ausführungsformen,
   ferner umfassend das Aufsputtern von Kohlenstoff und/oder Silizium und/oder von Metallen.
10. Verfahren nach einer der vorhergehenden Ausführungsformen,
   **dadurch gekennzeichnet,** dass in die kohlenstoffhaltige Schicht mittels Ionenimplantierung modifiziert wird.
11. Verfahren nach einer der vorhergehenden Ausführungsformen,
   **dadurch gekennzeichnet,** dass die kohlenstoffhaltige Schicht mit Oxidationsmitteln und/oder Reduktionsmitteln nachbehandelt wird, bevorzugt durch Behandeln der beschichteten Vorrichtung in oxidierender Säure oder Lauge chemisch modifiziert wird.
12. Verfahren nach einer der vorhergehenden Ausführungsformen,
   **dadurch gekennzeichnet,** dass die kohlenstoffhaltige Schicht mittels Lösemittel oder Lösemittelgemischen gereinigt wird.
13. Verfahren nach einer der vorhergehenden Ausführungsformen,
   **dadurch gekennzeichnet,** dass die Schritte a) und b) mehrfach durchgeführt werden, um eine kohlenstoffhaltige Multilayerbeschichtung zu erhalten, vorzugsweise mit unterschiedlichen Porositäten durch Vorstrukturierung der Polymerfilme oder Substrate oder geeignete oxidative Behandlung einzelner Schichten.
14. Verfahren nach einer der vorhergehenden Ausführungsformen,
   **dadurch gekennzeichnet,** dass in Schritt a) mehrere Polymerfilmschichten übereinander aufgebracht werden.
15. Verfahren nach einer der vorhergehenden Ausführungsformen,
   **dadurch gekennzeichnet,** dass die kohlenstoffhaltig beschichtete medizinische Vorrichtung mit mindestens einer zusätzlichen Schicht aus biologisch abbaubaren bzw. resorbierbaren Polymeren oder nicht-biologisch abbaubaren bzw. resorbierbaren Polymeren zumindest teilweise beschichtet wird.
16. Verfahren nach Ausführungsform 15,
   **dadurch gekennzeichnet,** dass die biologisch abbaubaren bzw. resorbierbaren Polymere ausgewählt sind aus Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymeren..
17. Verfahren nach einer der vorhergehenden Ausführungsformen,
   **dadurch gekennzeichnet,** dass die kohlenstoffhaltige Beschichtung auf der Vorrichtung mit mindestens einem Wirkstoff, mit Mikroorganismen oder lebenden Zellen beladen wird.
18. Verfahren nach Ausführungsform 17,
   **dadurch gekennzeichnet,** dass der mindestens eine Wirkstoff mittels Adsorption, Absorption, Physisorption, Chemisorption, kovalente Bindung, oder nicht-kovalente Bindung, elektrostatische Fixierung, oder Okklusion in Poren auf oder in der Beschichtung aufgebracht bzw. immobilisiert wird.
19. Verfahren nach einer der Ausführungsformen 17 oder 18,
   **dadurch gekennzeichnet,** dass der mindestens eine Wirkstoff im wesentlichen permanent auf oder in der Beschichtung immobilisiert ist.
20. Verfahren nach Ausführungsform 19,
   **dadurch gekennzeichnet,** dass der Wirkstoff anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, sowie pharmakologisch wirksame Stoffe oder Stoffgemische, Kombinationen dieser und dergleichen umfasst.
21. Verfahren nach einer der Ausführungsformen 17 oder 18,
   **dadurch gekennzeichnet,** dass der mindestens eine auf oder in der Beschichtung enthaltene Wirkstoff aus der Beschichtung kontrolliert freisetzbar ist.
22. Verfahren nach Ausführungsform 21,
   **dadurch gekennzeichnet,** dass der kontrolliert freisetzbare Wirkstoff anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, und dergleichen, sowie pharmakologisch wirksame Stoffe oder Stoffgemische umfasst.
23. Verfahren nach einer der Ausführungsformen 20 oder 22,
   **dadurch gekennzeichnet,** dass die pharmakologisch wirksamen Stoffe ausgewählt sind aus Heparin, synthetische Heparin-Analoga (z.B. Fondaparinux), Hirudin, Antithrombin III, Drotrecogin alpha; Fibrinolytica wie Alteplase, Plasmin, Lysokinasen, Faktor XIIa, Prourokinase, Urokinase, Anistreplase, Streptokinase; Thrombozytenaggregations-hemmer wie Acetylsalicylsäure, Ticlopidine, Clopidogrel, Abciximab, Dextrane; Corticosteroide wie Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone; sogenannte Non-Steroidal Anti-Inflammatory Drugs wie Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib; Zytostatika wie Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purin- oder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, und Antiöstrogene; Antiarrythmika, insbesondere Antiarrhythmika der Klasse I wie Antiarrhythmika vom Chinidintyp, z.B. Chinidin, Dysopyramid, Ajmalin, Prajmaliumbitartrat, Detajmiumbitartrat; Antiarrhythmika vom Lidocaintyp, z.B. Lidocain, Mexiletin, Phenytoin, Tocainid; Antiarrhythmika der Klasse I C, z.B. Propafenon, Flecainid(acetat); Antiarrhythmika der Klasse II, Betarezeptorenblocker wie Metoprolol, Esmolol, Propranolol, Metoprolol, Atenolol, Oxprenolol; Antiarrhythmika der Klasse III wie Amiodaron, Sotalol; Antiarrhythmika der Klasse IV wie Diltiazem, Verapamil, Gallopamil; andere Antiarrhythmika wie Adenosin, Orciprenalin, Ipratropiumbromid; Agenzien zur Stimulation der Angiogenese im Myokard wie Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC); Digitalisglykoside wie Acetyldigoxin/Metildigoxin, Digitoxin, Digoxin; Herzglykoside wie Ouabain, Proscillaridin; Antihypertonika wie zentral wirksame antiadrenerge Substanzen, z.B. Methyldopa, Imidazolinrezeptoragonisten; Kalciumkanalblocker vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker wie Prazosin, Urapidil, Doxazosin, Bunazosin, Terazosin, Indoramin; Vasodilatatoren wie Dihydralazin, Diisopropylamindichloracetat, Minoxidil, Nitroprussidnatrium; andere Antihypertonika wie Indapamid, Co-Dergocrinmesilat, Dihydroergotoxinmethansulfonat, Cicletanin, Bosentan, Fludrocortison; Phosphodiesterasehemmer wie Milrinon, Enoximon und Antihypotonika, wie insbesondere adrenerge und dopaminerge Substanzen wie Dobutamin, Epinephrin, Etilefrin, Norfenefrin, Norepinephrin, Oxilofrin, Dopamin, Midodrin, Pholedrin, Ameziniummetil; und partielle Adrenozeptor-Agonisten wie Dihydroergotamin; Fibronectin, Polylysine, Ethylenevinylacetate, inflammatorische Zytokine wie: TGFβ, PDGF, VEGF, bFGF, TNFα, NGF, GM-CSF, IGF-a, IL-1, IL-8, IL-6, Growth Hormone; sowie adhäsive Substanzen wie Cyanacrylate, Beryllium, Silica; und Wachstumsfaktoren (Growth Factor) wie Erythropoetin, Hormonen wie Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, Octreotid; Bone and Cartilage Stimulating Peptides, bone morphogenetic proteins (BMPs), insbesondere rekombinante BMP's wie z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride wie Dinatriumfluorophosphat, Natriumfluorid; Calcitonin, Dihydrotachystyrol; Wachstumsfaktoren und Zytokine wie Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs); Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, und Ethanol; ferner Silber(ionen), Titandioxid, Antibiotika und Antiinfektiva wie insbesondere β -Laktam-Antibiotika, z.B. β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine wie Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil; Aztreonam, Ertapenem, Meropenem; β-Lactamase-Inhibitoren wie Sulbactam, Sultamicillintosilat; Tetracycline wie Doxycyclin, Minocyclin, Tetracyclin, Chlortetracyclin, Oxytetracyclin; Aminoglykoside wie Gentamicin, Neomycin, Streptomycin, Tobramycin, Amikacin, Netilmicin, Paromomycin, Framycetin, Spectinomycin; Makrolidantibiotika wie Azithromycin, Clarithromycin, Erythromycin, Roxithromycin, Spiramycin, Josamycin; Lincosamide wie Clindamycin, Lincomycin, Gyrasehemmer wie Fluorochinolone wie Ciprofloxacin, Ofloxacin, Moxifloxacin, Norfloxacin, Gatifloxacin, Enoxacin, Fleroxacin, Levofloxacin; Chinolone wie Pipemidsäure; Sulfonamide, Trimethoprim, Sulfadiazin, Sulfalen; Glykopeptidantibiotika wieVancomycin, Teicoplanin; Polypeptidantibiotika wie Polymyxine wie Colistin, Polymyxin-B, Nitroimidazol-Derivate wie Metronidazol, Tinidazol; Aminochinolone wie Chloroquin, Mefloquin, Hydroxychloroquin; Biguanide wie Proguanil; Chininalkaloide und Diaminopyrimidine wie Pyrimethamin; Amphenicole wie Chloramphenicol; Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid; Virustatika wie Aciclovir, Ganciclovir, Famciclovir, Foscarnet, Inosin-(Dimepranol-4-acetamidobenzoat), Valganciclovir, Valaciclovir, Cidofovir, Brivudin; antiretrovirale Wirkstoffe wie nukleosidanaloge Reverse-Transkriptase-Hemmer und -Derivate, Lamivudin, Zalcitabin, Didanosin, Zidovudin, Tenofovir, Stavudin, Abacavir; nicht nukleosidanaloge Reverse-Transkriptase-Hemmer wie Amprenavir, Indinavir, Saquinavir, Lopinavir, Ritonavir, Nelfinavir; Amantadin, Ribavirin, Zanamivir, Oseltamivir und Lamivudin, sowie beliebige Kombinationen und Gemische davon.
24. Verfahren nach einer der Ausführungsformen 20 bis 23,
   **dadurch gekennzeichnet,** dass die pharmakologisch wirksamen Stoffe in Microcapsules, Liposomen, Nanocapsules, Nanopartikeln, Micellen, synthetischen Phospholipide, Gas-Dispersionen, Emulsionen, Mikroemulsionen, oder Nanospheres inkorporiert werden, die in den Poren oder an der Oberfläche der kohlenstoffhaltigen Schicht für eine spätere Freisetzung im Körper reversibel adsorbiert und/oder absorbiert werden.
25. Verfahren nach einer der vorhergehenden Ausführungsformen,
   **dadurch gekennzeichnet,** dass die implantierbare medizinische Vorrichtung ein Stent, bestehend aus einem Material ausgewählt aus der Gruppe aus rostfreiem Stahl, Platinhaltigen radiopaken Stahllegierungen, Kobaltlegierungen, Titanlegierungen, hochschmelzenden Legierungen auf Basis von Niob, Tantal, Wolfram und Molybdän, Edelmetallegierungen, Nitinollegierungen, sowie Magnesiumlegierungen und Mischungen der vorgenannten besteht.
26. Biokompatibel beschichtete, implantierbare medizinische Vorrichtung umfassend eine kohlenstoffhaltige Oberflächenbeschichtung, herstellbar nach einer der vorhergehenden Ausführungsformen.
27. Vorrichtung nach Ausführungsform 26, bestehend aus Metallen wie rostfreiem Stahl, Titan, Tantal, Platin, Nitinol, oder Nickel-Titan Legierung; Kohlefasern, Vollcarbonmaterial, Kohlenstoffkomposit, Keramik, Glas oder Glasfasern.
28. Vorrichtung nach Ausführungsform 26 oder 27,
   umfassend mehrere kohlenstoffhaltige Schichten, vorzugsweise mit unterschiedlichen Porositäten.
29. Vorrichtung nach einer der Ausführungsformen 26 bis 28,
   zusätzlich umfassend eine Beschichtung aus biologisch abbaubaren bzw. resorbierbaren Polymeren wie Kollagen, Albumin, Gelatin, Hyaluronsäure, Stärke, Cellulosen wie Methylcellulose, Hydroxypropylcellulose, Hydroxypropylmethyl-cellulose, Carboxymethylcellulose-Phthalat; Wachse, Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymere.
30. Vorrichtung nach einer der Ausfiihrungsformen 26 bis 28,
   zusätzlich umfassend eine Beschichtung aus nicht biologisch abbaubaren bzw. resorbierbaren Polymeren wie Poly(Ethylen-Vinylacetate), Silicone, Acrylpolymere wie Polyacrylsäure, Polymethylacrylsäure, Polyacrylcyanoacrylat; Polyethylene, Polypropylene, Polyamide, Polyurethane, Poly(Ester-Urethane), Poly(Ether-Urethane), Poly(Ester-Harnstoffe), Polyether (Poly(Ethylenoxid), Poly(Propylenoxid), Pluronics, Poly(Tetramethylenglycol); Vinylpolymere wie Polyvinylpyrrolidone, Poly(Vinylalkohole), oder Poly(vinylacetat-phtalat), sowie deren Copolymere.
31. Vorrichtung nach einer der Ausführungsformen 26 bis 30,
   ferner umfassend anionische oder kationischen oder amphotere Beschichtungen, wie z.B. Alginat, Carrageenan, Carboxymethylcellulose; Chitosan, Poly-L-Lysine; und/oder Phoshporylcholin.
32. Vorrichtung nach einer der Ausführungsformen 26 bis 31,
   **dadurch gekennzeichnet,** dass die kohlenstoffhaltige Schicht porös, vorzugsweise makroporös mit Porendurchmessern im Bereich von 0,1 bis 1000µm, und besonders bevorzugt nanoporös ist.
33. Vorrichtung nach einer der Ausführungsformen 26 bis 31,
   **dadurch gekennzeichnet,** dass die kohlenstoffhaltige Schicht nicht porös bzw. im wesentlichen geschlossenporig ist.
34. Vorrichtung nach einer der Ausführungsformen 26 bis 33,
   enthaltend einen oder mehrere Wirkstoffe wie in Ausführungsform 19 genannt.
35. Vorrichtung nach Ausführungsform 34,
   ferner umfassend eine die Freisetzung der Wirkstoffe beeinflussende Beschichtung ausgewählt aus pH-sensitiven und/oder Temperatursensitiven Polymeren und/oder biologisch aktiven Barrieren wie z.B. Enzymen.
36. Beschichteter Stent nach einer der Ausführungsformen 26 bis 35.
37. Beschichteter Stent nach Ausführungsform 36, ausgewählt aus rostfreiem Stahl, vorzugsweise Fe-18Cr-14Ni-2.5Mo ("316LVM" ASTM F138), Fe-21Cr-10Ni-3.5Mn-2.5Mo (ASTM F 1586), Fe-22Cr-13Ni-5Mn (ASTM F 1314), Fe-23Mn-21Cr-1Mo-1N (Nickelfreierrostfreier Stahl); aus Kobaltlegierungen, vorzugsweise Co-20Cr-15W-10Ni ("L605" ASTM F90), Co-20Cr-35Ni-10Mo ("MP35N" ASTM F 562), Co-20Cr-16Ni-16Fe-7Mo ("Phynox" ASTM F 1058); aus Titanlegierungen wie CP Titanium (ASTM F 67, Grade 1), Ti-6Al-4V (Alpha/beta ASTM F 136), Ti-6Al-7Nb (alpha/beta ASTM F1295), Ti-15Mo (beta grade ASTM F2066); aus Edelmetalllegierungen, insbesondere Iridiumhaltige Legierungen wie Pt-10Ir; Nitinollegierungen wie martensitische, superelastische und kaltbearbeitete Nitinole; sowie Magnesiumlegierungen wie Mg-3A1-1Z; sowie mindestens einer kohlenstoffhaltigen Oberflächenschicht.
38. Beschichtete Herzklappe nach einer der Ausführungsformen 26 bis 35.
39. Vorrichtung nach einer der Ausführungsformen 26 bis 35,
   in Form einer orthopädischen Knochen- oder Gelenkprothese, eines Knochensubstituts oder eines Wirbelkörperersatzmittels im Brust- oder Lendenbereich der Wirbelsäule.
40. Vorrichtung nach einer der Ausführungsformen 26 bis 35,
   in Form eines subkutanen und/oder intramuskulären Implantats zur kontrollierten Wirkstoffabgabe.

### Zusammenfassung

Es werden implantierbare medizinische Vorrichtungen mit biokompatiblen Beschichtungen sowie Verfahren zu deren Herstellung beschrieben. Insbesondere betrifft die vorliegende Erfindung mit einer kohlenstoffhaltigen Schicht beschichtete medizinische implantierbare Vorrichtungen, die durch mindestens partielles Beschichten der Vorrichtung mit einem Polymerfilm und Erhitzen des Polymerfilms in einer Atmosphäre, die im Wesentlichen frei von Sauerstoff ist, auf Temperaturen im Bereich von 200°C bis 2500°C hergestellt werden, wobei eine kohlenstoffhaltige Schicht auf der implantierbaren medizinischen Vorrichtung erzeugt wird.

## Patentansprüche

1. Biokompatibel beschichtete, implantierbare, medizinische Gefäßendoprothese, umfassend eine poröse, kohlenstoffhaltige Oberflächenbeschichtung, herstellbar nach einem Verfahren mit den Schritten
a) mindestens partielles Beschichten einer zu beschichtenden Gefäßendoprothese mit einem Polymerfilm mittels eines geeigneten Beschichtungs- bzw. Auftragungsverfahrens;
b) Erhitzen des Polymerfilms in einer Atmosphäre, die im Wesentlichen frei von Sauerstoff ist, auf Temperaturen im Bereich von 200°C bis 2500°C, zur Erzeugung einer kohlenstoffhaltigen Schicht auf dem Implantat,
c) Nachbehandlung der kohlenstoffhaltigen Schicht mit Oxidationsmitteln.

2. Gefäßendoprothese nach Anspruch 1,
wobei die Gefäßendoprothese ausgewählt ist aus einem Stent, einem Koronarstent, einem intravaskularen Stent und einem peripheren Stent.

3. Gefäßendoprothese nach Anspruch 1 oder 2,
wobei die zu beschichtende Gefäßendoprothese aus Metall, wie rostfreiem Stahl, Titan, Tantal, Platin, Nitinol, Nitinollegierungen, Platin-haltigen radiopaken Stahllegierungen, Kobaltlegierungen, Titanlegierungen, Magnesiumlegierungen, hochschmelzenden Legierungen auf Basis von Niob, Tantal, Wolfram und Molybdän, Edelmetallegierungen oder Nickel-Titan Legierung; Kohlefasern, Vollcarbonmaterial, Kohlenstoffkomposit, Keramik, Glas oder Glasfasern besteht.

4. Gefäßendoprothese nach einem der vorhergehenden Ansprüche,
wobei die zu beschichtende Gefäßendoprothese aus rostfreiem Stahl, insbesondere Fe-18Cr-14Ni-2.5Mo ("316LVM" ASTM F138), Fe-21Cr-10Ni-3.5Mn-2.5Mo (ASTM F 1586), Fe-22Cr-13Ni-5Mn (ASTM F 1314), Fe-23Mn-21Cr-1Mo-1N (Nickelfreier rostfreier Stahl); aus Kobaltlegierungen, insbesondere Co-20Cr-15W-10Ni ("L605" ASTM F90), Co-20Cr-35Ni-10Mo ("MP35N" ASTM F 562), Co-20Cr-16Ni-16Fe-7Mo ("Phynox" ASTM F 1058); aus Titanlegierungen, insbesondere CP Titanium (ASTM F 67, Grade 1), Ti-6Al-4V (Alpha/beta ASTM F 136), Ti-6Al-7Nb (alpha/beta ASTM F1295), Ti-15Mo (beta grade ASTM F2066); aus Edelmetalllegierungen, insbesondere iridiumhaltigen Legierungen, Pt-10Ir; aus Nitinollegierungen, martensitischen, superelastischen oder kaltbearbeiteten Nitinolen; sowie aus Magnesiumlegierungen oder Mg-3A1-1Z besteht.

5. Gefäßendoprothese nach einem der vorhergehenden Ansprüche,
umfassend mehrere kohlenstoffhaltige Schichten, hergestellt durch mehrfaches Durchführen der Schritte a) und b).

6. Gefäßendoprothese nach einem der vorhergehenden Ansprüche,
wobei die kohlenstoffhaltige Schicht porös, vorzugsweise makroporös mit Porendurchmessern im Bereich von 0,1 bis 1000µm, und besonders bevorzugt nanoporös ist.

7. Gefäßendoprothese nach einem der vorhergehenden Ansprüche,
wobei die kohlenstoffhaltige Beschichtung auf der Gefäßendoprothese mit mindestens einem Wirkstoff, mit Mikroorganismen oder lebenden Zellen beladen ist.

8. Gefäßendoprothese nach Anspruch 7,
wobei der Wirkstoff in Microcapsules, Liposomen, Nanocapsules, Nanopartikeln, Micellen, synthetischen Phospholipiden, Gas-Dispersionen, Emulsionen, Mikroemulsionen, oder Nanospheres inkorporiert ist, die in Poren oder an der Oberfläche der kohlenstoffhaltigen Schicht für eine spätere Freisetzung im Körper reversibel adsorbiert und/oder absorbiert sind.

9. Gefäßendoprothese nach einem der Ansprüche 7 oder 8,
wobei der Wirkstoff anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, sowie pharmakologisch wirksame Stoffe oder Stoffgemische, Kombinationen dieser und dergleichen umfasst.

10. Gefäßendoprothese nach einem der Ansprüche 7 bis 9,
ferner umfassend eine die Freisetzung der Wirkstoffe beeinflussende Beschichtung ausgewählt aus pH-sensitiven und/oder Temperatur-sensitiven Polymeren und/oder biologisch aktiven Barrieren.

11. Gefäßendoprothese nach einem der Ansprüche 7 bis 10,
wobei der mindestens eine auf oder in der Beschichtung enthaltene Wirkstoff aus der Beschichtung kontrolliert freisetzbar ist.

12. Gefäßendoprothese nach einem der vorhergehenden Ansprüche,
wobei der Polymerfilm Homo- oder Copolymere von aliphatischen oder aromatischen Polyolefinen wie Polyethylen, Polypropylen, Polybuten, Polyisobuten, Polypenten; Polybutadien; Polyvinyle wie Polyvinylchorid oder Polyvinylalkohol, Poly(meth)acrylsäure, Polyacrylcyanoacrylat; Polyacrylnitril, Polyamid, Polyester, Polyurethan, Polystyrol, Polytetrafluorethylen; Polymeren wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Wachse, Paraffinwachse, Fischer-Tropsch-Wachse; Kasein, Dextrane, Polysaccharide, Fibrinogen, Poly(D,L-Lactide), Poly(D,L-Lactide-Co-Glycolide), Polyglycolide, Polyhydroxybutylate, Polyalkylcarbonate, Polyorthoester, Polyester, Polyhydroxyvalerinsäure, Polydioxanone, Polyethylenterephtalat, Polymalatsäure, Polytartronsäure, Polyanhydride, Polyphosphazene, Polyaminosäuren; Polyethylenvinylacetat, Silikone; Poly(Ester-Urethane), Poly(Ether-Urethane), Poly(Ester-Harnstoffe), Polyether wie Polyethylenoxid, Polypropylenoxid, Pluronics, Polytetramethylenglycol; Polyvinylpyrrolidon, Poly(vinyl-acetat-phatalat), sowie deren Copolymere, Mischungen und Kombinationen dieser Homo- oder Copolymere umfasst.

13. Gefäßendoprothese nach einem der vorhergehenden Ansprüche,
wobei der Polymerfilm Alkydharz, Chlorkautschuk, Epoxidharz, Acrylatharz, Phenolharz, Aminharz, Melaminharz, Alkylphenolharze, epoxidierte aromatische Harze, Ölbasis, Nitrobasis, Polyester, Polyurethan, Teer, teerartige Materialien, Teerpech, Bitumen, Stärke, Zellulose, Wachse, Schellack, organische Materialien aus nachwachsenden Rohstoffen oder Kombinationen davon umfasst.

14. Gefäßendoprothese nach einem der vorhergehenden Ansprüche,
wobei die kohlenstoffhaltige Schicht durch Behandeln der beschichteten Vorrichtung in oxidierender Säure oder Lauge chemisch modifiziert wird.

15. Gefäßendoprothese nach einem der vorhergehenden Ansprüche,
wobei die Nachbehandlung mit Luft bei einer Temperatur im Bereich von 40 °C bis 1000°C durchgeführt wird.
